(19) Europäisches Patentamt
European Patent Office
Office européen des brevets

(11) EP 4 399 269 B1

(12) **EUROPEAN PATENT SPECIFICATION**

(45) Date of publication and mention of the grant of the patent:
**05.11.2025 Bulletin 2025/45**

(21) Application number: **22778161.4**

(22) Date of filing: **07.09.2022**

(51) International Patent Classification (IPC):
***C11D 3/20*** (2006.01) ***C11D 3/00*** (2006.01)
***C11D 11/00*** (2006.01)

(52) Cooperative Patent Classification (CPC):
**C11D 3/2093; C11D 3/0084;** C11D 2111/12

(86) International application number:
**PCT/US2022/042756**

(87) International publication number:
**WO 2023/038971 (16.03.2023 Gazette 2023/11)**

(54) **PHENOLIC COMPOSITIONS FOR MALODOR REDUCTION**

PHENOLZUSAMMENSETZUNGEN ZUR VERMINDERUNG VON SCHLECHTEM GERUCH

COMPOSITIONS PHÉNOLIQUES POUR LA RÉDUCTION DES MAUVAISES ODEURS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **09.09.2021 US 202163242214 P**

(43) Date of publication of application:
**17.07.2024 Bulletin 2024/29**

(73) Proprietor: **Milliken & Company**
**Spartanburg, South Carolina 29303 (US)**

(72) Inventors:
- **FREUND, Wesley**
  **Spartanburg, South Carolina 29303 (US)**
- **L'AMOREAUX, Nicholas**
  **Spartanburg, South Carolina 29303 (US)**

(74) Representative: **Elkington and Fife LLP**
**Prospect House**
**8 Pembroke Road**
**Sevenoaks, Kent TN13 1XR (GB)**

(56) References cited:
**CN-A- 106 588 663**
**JP-A- 2011 130 986**
**US-A1- 2021 139 816**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

## FIELD OF THE INVENTION

The present disclosure relates to phenolic compositions for use as antioxidants in fabric treatment compositions for reducing malodor.

## BACKGROUND

Malodor is a lingering issue in laundry. There are a number of sources of malodor causing materials on textiles. One source of malodor comes from the fact that as consumers move toward the use of less water, shorter wash cycles and lower wash water temperatures, soils are not as efficiently removed from the clothes during the wash cycle and remain on the surface of the fabrics. These soils that remain on the fabric service may consist of human sebum and other oils that are prone to autoxidation and breakdown into volatile, malodorous compounds.
Manufacturers are continuously seeking ways to reduce this malodor. One approach involves the use of antioxidants in the laundry that are deposited onto clothing during the laundering. These antioxidants reduce the rate of autoxidation of the oils, and in turn prevent the generation of malodorous compounds. JP 2011130986 A discloses a laundry deodorizer composition comprising quaternary surfactant and Irganox 245.
There remains a need for improved antioxidant compositions for reducing malodor in fabrics and textiles. There remains a need for improved methods of preventing oxidation of sebum and other oils in fabric and textiles. There remains a need for improved antioxidant compositions that effectively reduce and block malodor over increased periods of time. There remains a need for improved antioxidant compositions that effectively reduce and block malodor when applied at lower concentrations. There remains a need for improved antioxidant compositions that are more readily formulated into detergent compositions. There remains a need for improved antioxidant compositions that are more readily transferred to fabrics and textiles when laundered.

## SUMMARY

In accordance with the purposes of the disclosed materials and methods, as embodied and broadly described herein, the disclosed subject matter, in one aspect, relates to compositions and methods of making and using compositions.
Additional advantages will be set forth in part in the description that follows, and in part will be obvious from the description, or may be learned by practice of the aspects described below. The invention is defined in the appended claims. It is to be understood that both the foregoing general description and the following detailed description are exemplary and explanatory only and are not restrictive.
The details of one or more embodiments are set forth in the descriptions below. Other features, objects, and advantages will be apparent from the description and from the claims.

## DETAILED DESCRIPTION

Before the present methods and systems are disclosed and described, it is to be understood that the methods and systems are not limited to specific synthetic methods, specific components, or to particular compositions. It is also to be understood that the terminology used herein is for the purpose of describing particular embodiments only and is not intended to be limiting.
As used in the specification and the appended claims, the singular forms "a," "an" and "the" include plural referents unless the context clearly dictates otherwise. Ranges may be expressed herein as from "about" one particular value, and/or to "about" another particular value. When such a range is expressed, another embodiment includes¬ from the one particular value and/or to the other particular value. Similarly, when values are expressed as approximations, by use of the antecedent "about," it will be understood that the particular value forms another embodiment. It will be further understood that the endpoints of each of the ranges are significant both in relation to the other endpoint, and independently of the other endpoint.
"Optional" or "optionally" means that the subsequently described event or circumstance may or may not occur, and that the description includes instances where said event or circumstance occurs and instances where it does not.
Throughout the description and claims of this specification, the word "comprise" and variations of the word, such as "comprising" and "comprises," means "including but not limited to," and is not intended to exclude, for example, other additives, components, integers, or steps. "Exemplary" means "an example of" and is not intended to convey an indication of a preferred or ideal embodiment. "Such as" is not used in a restrictive sense, but for explanatory purposes.
Disclosed are components that can be used to perform the disclosed methods and systems. These and other

components are disclosed herein, and it is understood that when combinations, subsets, interactions, groups, etc. of these components are disclosed that while specific reference of each various individual and collective combinations and permutation of these may not be explicitly disclosed, each is specifically contemplated and described herein, for all methods and systems. This applies to all aspects of this application including, but not limited to, steps in disclosed methods. Thus, if there are a variety of additional steps that can be performed it is understood that each of these additional steps can be performed with any specific embodiment or combination of embodiments of the disclosed methods.

As used herein the phrase "fabric treatment composition" includes compositions and formulations designed for treating fabrics, including garments, or other textiles. Such compositions include, but are not limited to, laundry cleaning compositions and detergents, fabric softening compositions, fabric enhancing compositions, fabric freshening compositions, laundry prewash, laundry pretreat, laundry additives, spray products, dry cleaning agent or composition, laundry wash additives, post-rinse fabric treatments, ironing aid, unit dose formulation, delayed delivery formulation, detergent contained on or in a porous substrate or nonwoven sheet, and other suitable forms that may be apparent to one skilled in the art in view of the teachings herein. Such compositions may be used as a pre-laundering treatment, a post-laundering treatment, or may be added during the wash cycle of the laundering operation.

As used herein, "liquid" includes free-flowing liquids, as well as pastes, gels, foams, and mousses. Non-limiting examples of liquids include light-duty and heavy-duty liquid detergent compositions, fabric enhancers, detergent gels commonly used for laundry, bleach, and laundry additives. Gases, e.g., suspended bubbles, or solids, e.g., particles, may be included within the liquids.

As used herein, a "granule" and a "particle" refer to a volume of solid, or sufficiently solid, material that has finite mass. Granules and particles may be free-flowing or suspended within a secondary composition. Free-flowing particles may be similar to those commercially available under the tradename UNSTOPABLES® from The Procter & Gamble Company, Cincinnati, Ohio, United States.

The terms "substantially free of" or "substantially free from" may be used herein. This means that the indicated material is at the very minimum not deliberately added to the composition to form part of it, or, preferably, is not present at analytically detectable levels. It is meant to include compositions whereby the indicated material is present only as an impurity in one of the other materials deliberately included. The indicated material may be present, if at all, at a level of less than 10%, or less than 1%, or less than 0.1%, or less than 0.01%, or even 0%, by weight of the composition.

As used herein, the phrases "sufficiently solid" and "solid" mean the material is capable of maintaining its shape without significant deformation when free-standing at room temperature. A "solid" as used herein may include, but is not limited to, granules, particles, powders, agglomerates, micro-capsules, flakes, noodles, pearlized balls, and mixtures thereof.

As used herein, the phrase "water-soluble", "water-soluble material," "water-soluble carrier material," means that the material or carrier material is soluble or dispersible in water, and preferably has a water-solubility of at least 50%, preferably at least 75% or even at least 95%, as measured by the method set out hereafter using a glass-filter with a maximum pore size of 20 microns: 50 grams±0.1 gram of the material and/or carrier material is added in a preweighed 400 mL beaker and 245 mL±1 mL of distilled water is added. This is stirred vigorously on a magnetic stirrer set at 600 rpm, for 30 minutes. Then, the mixture is filtered through a sintered-glass filter with a pore size as defined above (max. 20 micron). The steps are performed at ambient conditions. "Ambient conditions" as used herein means 23° C.±1.0° C. and a relative humidity of 50%±2%. The water is dried off from the collected filtrate by any conventional method, and the weight of the remaining material is determined (which is the dissolved or dispersed fraction). Then, the percentage solubility or dispersibility can be calculated.

Unless otherwise noted, all component/ingredient or composition levels are in reference to the active portion of that component/ingredient or composition, and are exclusive of impurities, for example, residual solvents or by-products, which may be present in commercially available sources of such components/ingredients or compositions.

The invention concerns laundry care compositions comprising surfactant and phenolic compositions for use as antioxidants in fabric treatment compositions. The phenolic compositions include at least a compound of Formula (I) and a compound of Formula (II):

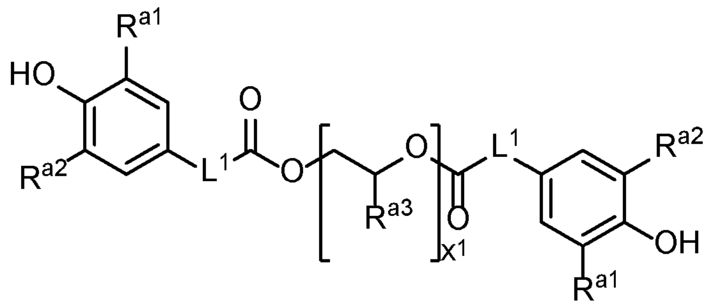

[Formula (I)],

wherein $L^1$ is selected from null, $-CH_2-$, $-CH_2CH_2-$, and $-CH_2CH_2CH_2-$,
$x^1$ is an integer from 2-50;
$R^{a1}$ is a $C_{1-8}$alkyl group;
$R^{a2}$ is a $C_{1-8}$alkyl group;
each $R^{a3}$ is independently selected from the group consisting of H, $CH_3$, and $CH_2CH_3$;

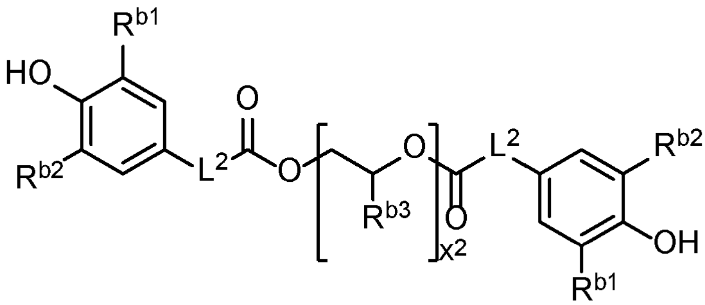

[Formula (II)],

wherein $L^2$ is selected from null, $-CH_2-$, $-CH_2CH_2-$, and $-CH_2CH_2CH_2-$,
$x^2$ is an integer from 2-50; provided that $x^1$ and $x^2$ are not the same;
$R^{b1}$ is a $C_{1-8}$alkyl group;
$R^{b2}$ is a $C_{1-8}$alkyl group; and
each $R^{b3}$ is independently selected from the group consisting of H, $CH_3$, and $CH_2CH_3$.

In the context of Formula (I) and Formula (II) and the formula for the other phenolic compounds that follow, the term "null" is meant to convey that the phenol moiety is directly bonded to the carbonyl carbon atom *via* the carbon atom of the phenol moiety that is located in the *para-* position relative to the hydroxy group of the phenol moiety. The use of brackets for the $-[CH_2-CHR^3-O]-$ group does not imply that each $R^3$ group is necessarily the same. Unless specified to the contrary, a given compound can have the same or different $R^3$ substituents at each repeating unit of the chain. For example, in some embodiments of the compound of Formula (I) x=4. In some such embodiments, $R^{a3}$ can in each case be hydrogen, while in other cases two $R^{a3}$ groups can be hydrogen, and the other two $R^{a3}$ groups can be methyl.

Without wishing to be bound by theory, it is believed the use of multiple phenolic antioxidants impart favorable antioxidant strength, as well as stability, relative to the use of a single phenolic compound as an antioxidant.

In some embodiments, the phenolic compositions can further include a compound of Formula (III):

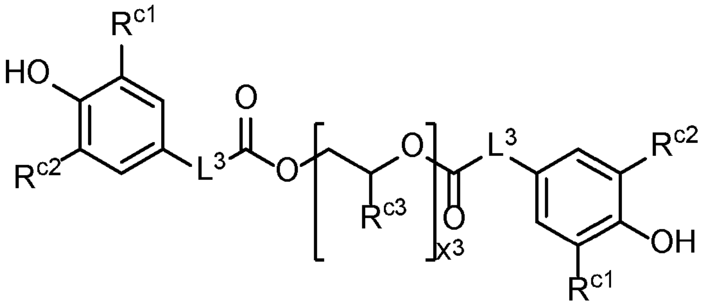

[Formula (III)],

wherein $L^3$ is selected from null, $-CH_2-$, $-CH_2CH_2-$, and $-CH_2CH_2CH_2-$,
$x^3$ is an integer from 2-50; provided that none of $x^1$, $x^2$, and $x^3$ are the same;
$R^{c1}$ is a $C_{1-8}$alkyl group;
$R^{c2}$ is a $C_{1-8}$alkyl group; and
each $R^{c3}$ is independently selected from the group consisting of H, $CH_3$, and $CH_2CH_3$.

In some embodiments, the phenolic compositions can further include a compound of Formula (IV):

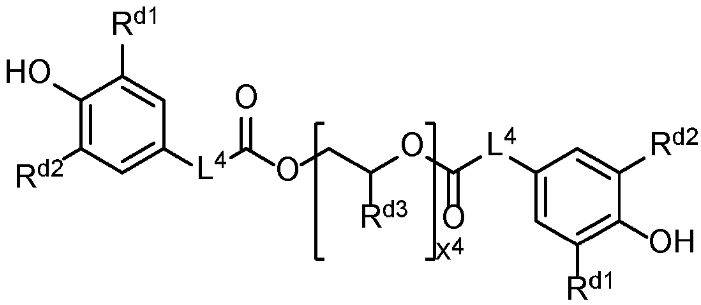

[Formula (IV)],

wherein $L^4$ is selected from null, $-CH_2-$, $-CH_2CH_2-$, and $-CH_2CH_2CH_2-$,
$x^4$ is an integer from 2-50; provided that none of $x^1$, $x^2$, $x^3$, and $x^4$ are the same;
$R^{d1}$ is a $C_{1-8}$alkyl group;
$R^{d2}$ is a $C_{1-8}$alkyl group; and
each $R^{d3}$ is independently selected from the group consisting of H, $CH_3$, and $CH_2CH_3$.

In some embodiments, the phenolic compositions can further include a compound of Formula (V):

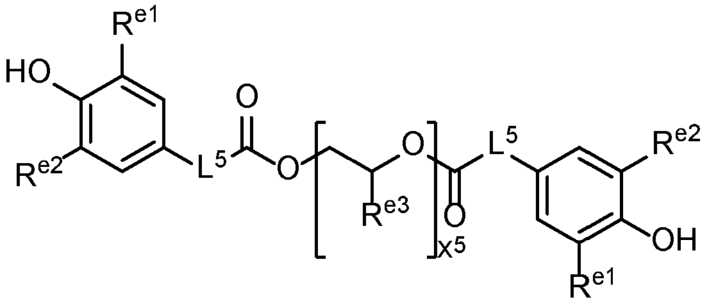

[Formula (V)],

wherein $L^5$ is selected from null, $-CH_2-$, $-CH_2CH_2-$, and $-CH_2CH_2CH_2-$,
$x^5$ is an integer from 2-50; provided that none of $x^1$, $x^2$, $x^3$, $x^4$, and $x^5$ are the same;
$R^{e1}$ is a $C_{1-8}$alkyl group;
$R^{e2}$ is a $C_{1-8}$alkyl group; and
each $R^{e3}$ is independently selected from the group consisting of H, $CH_3$, and $CH_2CH_3$.

In some embodiments, the phenolic compositions can further include a compound of Formula (VI):

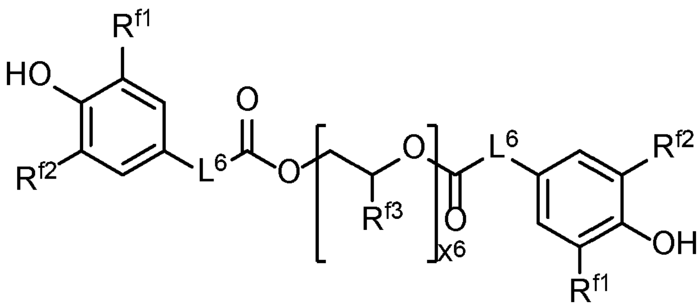

[Formula (VI)],

wherein $L^5$ is selected from null, $-CH_2-$, $-CH_2CH_2-$, and $-CH_2CH_2CH_2-$,
$x^6$ is an integer from 2-50; provided that none of $x^1$, $x^2$, $x^3$, $x^4$, $x^5$, and $x^6$ are the same;
$R^{f1}$ is a $C_{1-8}$alkyl group;
$R^{f2}$ is a $C_{1-8}$alkyl group; and
each $R^{f3}$ is independently selected from the group consisting of H, $CH_3$, and $CH_2CH_3$.

In some embodiments, the phenolic compositions can further include a compound of Formula (VII):

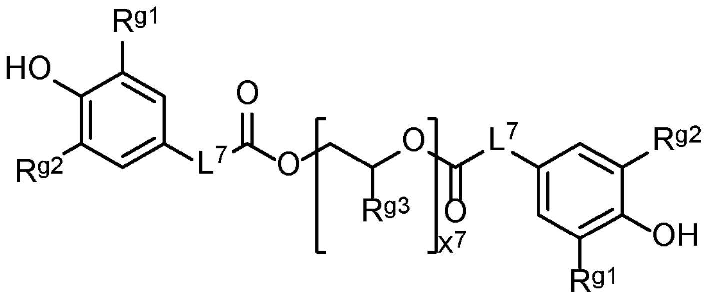

[Formula (VII)],

wherein $L^5$ is selected from null, $-CH_2-$, $-CH_2CH_2-$, and $-CH_2CH_2CH_2-$,
$x^7$ is an integer from 2-50; provided that none of $x^1$, $x^2$, $x^3$, $x^4$, $x^5$, $x^6$, and $x^7$ are the same;
$R^{g1}$ is a $C_{1-8}$alkyl group;
$R^{g2}$ is a $C_{1-8}$alkyl group; and
each $R^{g3}$ is independently selected from the group consisting of H, $CH_3$, and $CH_2CH_3$.

In some embodiments, the phenolic compositions can further include a compound of Formula (VIII):

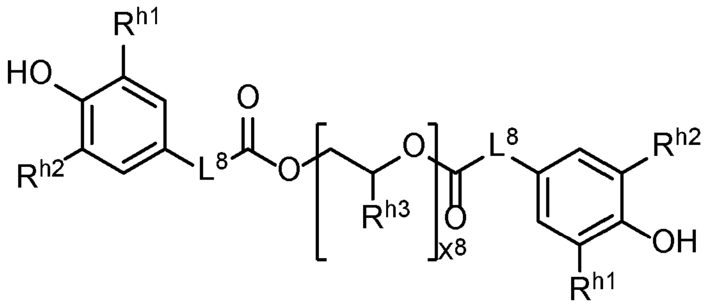

[Formula (VIII)],

wherein $L^5$ is selected from null, $-CH_2-$, $-CH_2CH_2-$, and $-CH_2CH_2CH_2-$,
$x^8$ is an integer from 2-50; provided that none of $x^1$, $x^2$, $x^3$, $x^4$, $x^5$, $x^6$, $x^7$, and $x^8$ are the same;
$R^{h1}$ is a $C_{1-8}$alkyl group;
$R^{h2}$ is a $C_{1-8}$alkyl group; and
each $R^{h3}$ is independently selected from the group consisting of H, $CH_3$, and $CH_2CH_3$.

In some embodiments, it is preferred that each of the L substituents (i.e., $L^1$, $L^2$, $L^3$, $L^4$, $L^5$, $L^6$, $L^7$, and $L^8$) is the same. For example, each L substituent can be null, or each L substituent can be $-CH_2-$, or each L substituent can be $-CH_2CH_2-$, or each L substituent can be - $CH_2CH_2CH_2-$. In preferred embodiments, each L substituent is $-CH_2CH_2-$.

In certain embodiments, it is preferred that the $R^1$ substituents (i.e., $R^{a1}$, $R^{b1}$, $R^{c1}$, $R^{d1}$, $R^{e1}$, $R^{f1}$, $R^{g1}$, and $R^{h1}$) are not the same as the $R^2$ substituents (i.e., $R^{a2}$, $R^{b2}$, $R^{c2}$, $R^{d2}$, $R^{e2}$, $R^{f2}$, $R^{g2}$, and $R^{h2}$). For example, each of the $R^1$ substituents can be a tert-butyl group, while each of the $R^2$ substituents can be a linear $C_{1-8}$alkyl group (i.e., methyl, ethyl, n-propyl, n-butyl, n-pentyl, n-hexyl, n-heptyl, or n-octyl). In certain embodiments, it is preferred than each of the $R^1$ substituents is a tert-butyl group, and each of the $R^2$ substituents is a methyl group, or each of the $R^2$ substituents is an ethyl group.

In some embodiments, it is preferred that each of the $R^3$ substituents (i.e., $R^{a3}$, $R^{b3}$, $R^{c3}$, $R^{d3}$, $R^{e3}$, $R^{f3}$, $R^{g3}$, and $R^{h3}$) are the same. For example, each of the $R^3$ substituents can be H, or each of the $R^3$ substituents can be $CH_3$. In other embodiments, at least one of the $R^3$ substituents is H and at least one of the $R^3$ substituents is $CH_3$.

For embodiments in which each of the $R^1$ substituents are the same, each of the $R^2$ substituents are the same,

each of the $R^3$ substituents are the same, and each of the L substituents are the same, the phenolic compositions can be described as a plurality of compounds having the general formula:

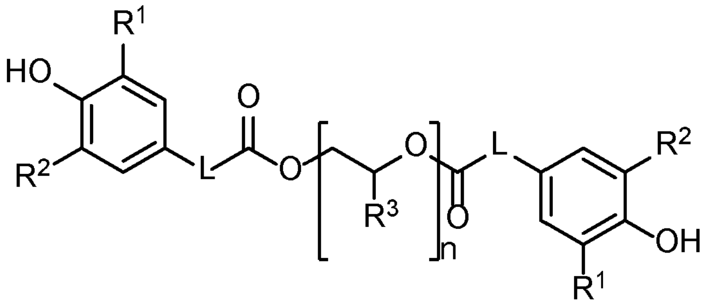

,

wherein L is selected from null, $-CH_2-$, $-CH_2CH_2-$, and $-CH_2CH_2CH_2-$,
$R^1$ is a $C_{1-8}$alkyl group;
$R^2$ is a $C_{1-8}$alkyl group;
$R^3$ is H, $CH_3$, or $CH_2CH_3$; and
n is greater than 2 and less than 50, preferably greater than 3 and less than 50, greater than 4 and less than 25, greater than 4 and less than 10, greater than 5 and less than 15, greater than 8 and less than 20, or greater than 12 and less than 25.

The 'n' value of a given phenolic composition may be determined by HPLC analysis with a UV/Vis detector. This type of analysis is the preferred method to quantify the levels of each component of the polymeric distribution, as it will give the most accurate, reproducible results with phenolic compositions of this type. In other embodiments, mass spectrometry data may also be used.

In some embodiments, the compound of Formula I is characterized by $x^1$=4, the compound of Formula II is characterized by $x^2$=3. The compound of Formula I may be present in the phenolic composition in an amount from 15-75%, relative to the total mass of the phenolic composition, and the compound of Formula II may be present in an amount from 15-75%, relative to the total mass of the phenolic composition.

In certain embodiments, the compound of Formula I is characterized by $x^1$=4, the compound of Formula II is characterized by $x^2$=3, and the compound of Formula III is characterized by $x^3$ = 5. The compound of Formula I may be present in the phenolic composition in an amount from 15-75%, relative to the total mass of the phenolic composition, the compound of Formula II may be present in an amount from 15-75%, relative to the total mass of the phenolic composition, and the compound of Formula III may be present in an amount from 15-75%, relative to the total mass of the phenolic composition.

In some embodiments, the compound of Formula I is characterized by $x^1$=5, the compound of Formula II is characterized by $x^2$=4. The compound of Formula I may be present in the phenolic composition in an amount from 15-75%, relative to the total mass of the phenolic composition, and the compound of Formula II may be present in an amount from 15-75%, relative to the total mass of the phenolic composition.

In certain embodiments, the compound of Formula I is characterized by $x^1$=5, the compound of Formula II is characterized by $x^2$=4, and the compound of Formula III is characterized by $x^3$ = 6. The compound of Formula I may be present in the phenolic composition in an amount from 15-75%, relative to the total mass of the phenolic composition, the compound of Formula II may be present in an amount from 15-75%, relative to the total mass of the phenolic composition, and the compound of Formula III may be present in an amount from 15-75%, relative to the total mass of the phenolic composition.

In some embodiments, the compound of Formula I is characterized by $x^1$=6, the compound of Formula II is characterized by $x^2$=5. The compound of Formula I may be present in the phenolic composition in an amount from 15-75%, relative to the total mass of the phenolic composition, and the compound of Formula II may be present in an amount from 15-75%, relative to the total mass of the phenolic composition.

In certain embodiments, the compound of Formula I is characterized by $x^1$=6, the compound of Formula II is characterized by $x^2$=5, and the compound of Formula III is characterized by $x^3$ = 7. The compound of Formula I may be present in the phenolic composition in an amount from 15-75%, relative to the total mass of the phenolic composition, the compound of Formula II may be present in an amount from 15-75%, relative to the total mass of the phenolic composition, and the compound of Formula III may be present in an amount from 15-75%, relative to the total mass of the phenolic composition.

In some embodiments, the compound of Formula I is characterized by $x^1$=7, the compound of Formula II is

characterized by $x^2$=6. The compound of Formula I may be present in the phenolic composition in an amount from 15-75%, relative to the total mass of the phenolic composition, and the compound of Formula II may be present in an amount from 15-75%, relative to the total mass of the phenolic composition.

In certain embodiments, the compound of Formula I is characterized by $x^1$=7, the compound of Formula II is characterized by $x^2$=6, and the compound of Formula III is characterized by $x^3$ = 8. The compound of Formula I may be present in the phenolic composition in an amount from 15-75%, relative to the total mass of the phenolic composition, the compound of Formula II may be present in an amount from 15-75%, relative to the total mass of the phenolic composition, and the compound of Formula III may be present in an amount from 15-75%, relative to the total mass of the phenolic composition.

In some embodiments, the compound of Formula I is characterized by $x^1$=8, the compound of Formula II is characterized by $x^2$=7. The compound of Formula I may be present in the phenolic composition in an amount from 15-75%, relative to the total mass of the phenolic composition, and the compound of Formula II may be present in an amount from 15-75%, relative to the total mass of the phenolic composition.

In certain embodiments, the compound of Formula I is characterized by $x^1$=8, the compound of Formula II is characterized by $x^2$=7, and the compound of Formula III is characterized by $x^3$ = 9. The compound of Formula I may be present in the phenolic composition in an amount from 15-75%, relative to the total mass of the phenolic composition, the compound of Formula II may be present in an amount from 15-75%, relative to the total mass of the phenolic composition, and the compound of Formula III may be present in an amount from 15-75%, relative to the total mass of the phenolic composition.

In some embodiments, the compound of Formula I is characterized by $x^1$=9, the compound of Formula II is characterized by $x^2$=7. The compound of Formula I may be present in the phenolic composition in an amount from 15-75%, relative to the total mass of the phenolic composition, and the compound of Formula II may be present in an amount from 15-75%, relative to the total mass of the phenolic composition.

In certain embodiments, the compound of Formula I is characterized by $x^1$=9, the compound of Formula II is characterized by $x^2$=7, and the compound of Formula III is characterized by $x^3$ = 10. The compound of Formula I may be present in the phenolic composition in an amount from 15-75%, relative to the total mass of the phenolic composition, the compound of Formula II may be present in an amount from 15-75%, relative to the total mass of the phenolic composition, and the compound of Formula III may be present in an amount from 15-75%, relative to the total mass of the phenolic composition.

In some embodiments, the compound of Formula I is characterized by $x^1$=10, the compound of Formula II is characterized by $x^2$=9 The compound of Formula I may be present in the phenolic composition in an amount from 15-75%, relative to the total mass of the phenolic composition, and the compound of Formula II may be present in an amount from 15-75%, relative to the total mass of the phenolic composition.

In certain embodiments, the compound of Formula I is characterized by $x^1$=10, the compound of Formula II is characterized by $x^2$=9, and the compound of Formula III is characterized by $x^3$ = 11. The compound of Formula I may be present in the phenolic composition in an amount from 15-75%, relative to the total mass of the phenolic composition, the compound of Formula II may be present in an amount from 15-75%, relative to the total mass of the phenolic composition, and the compound of Formula III may be present in an amount from 15-75%, relative to the total mass of the phenolic composition.

In some embodiments, the compound of Formula I is characterized by $x^1$=11, the compound of Formula II is characterized by $x^2$=10. The compound of Formula I may be present in the phenolic composition in an amount from 15-75%, relative to the total mass of the phenolic composition, and the compound of Formula II may be present in an amount from 15-75%, relative to the total mass of the phenolic composition.

In certain embodiments, the compound of Formula I is characterized by $x^1$=11, the compound of Formula II is characterized by $x^2$=10, and the compound of Formula III is characterized by $x^3$ = 12. The compound of Formula I may be present in the phenolic composition in an amount from 15-75%, relative to the total mass of the phenolic composition, the compound of Formula II may be present in an amount from 15-75%, relative to the total mass of the phenolic composition, and the compound of Formula III may be present in an amount from 15-75%, relative to the total mass of the phenolic composition.

In some embodiments, the compound of Formula I is characterized by $x^1$=12, the compound of Formula II is characterized by $x^2$=11. The compound of Formula I may be present in the phenolic composition in an amount from 15-75%, relative to the total mass of the phenolic composition, and the compound of Formula II may be present in an amount from 15-75%, relative to the total mass of the phenolic composition.

In certain embodiments, the compound of Formula I is characterized by $x^1$=12, the compound of Formula II is characterized by $x^2$=11, and the compound of Formula III is characterized by $x^3$ = 13. The compound of Formula I may be present in the phenolic composition in an amount from 15-75%, relative to the total mass of the phenolic composition, the compound of Formula II may be present in an amount from 15-75%, relative to the total mass of the phenolic composition, and the compound of Formula III may be present in an amount from 15-75%, relative to the total mass of the phenolic

composition.

In some embodiments, the compound of Formula I is characterized by $x^1$=13, the compound of Formula II is characterized by $x^2$=12. The compound of Formula I may be present in the phenolic composition in an amount from 15-75%, relative to the total mass of the phenolic composition, and the compound of Formula II may be present in an amount from 15-75%, relative to the total mass of the phenolic composition.

In certain embodiments, the compound of Formula I is characterized by $x^1$=13, the compound of Formula II is characterized by $x^2$=12, and the compound of Formula III is characterized by $x^3$ = 14. The compound of Formula I may be present in the phenolic composition in an amount from 15-75%, relative to the total mass of the phenolic composition, the compound of Formula II may be present in an amount from 15-75%, relative to the total mass of the phenolic composition, and the compound of Formula III may be present in an amount from 15-75%, relative to the total mass of the phenolic composition.

In some embodiments, the compound of Formula I is characterized by $x^1$=14, the compound of Formula II is characterized by $x^2$=13. The compound of Formula I may be present in the phenolic composition in an amount from 15-75%, relative to the total mass of the phenolic composition, and the compound of Formula II may be present in an amount from 15-75%, relative to the total mass of the phenolic composition.

In certain embodiments, the compound of Formula I is characterized by $x^1$=14, the compound of Formula II is characterized by $x^2$=13, and the compound of Formula III is characterized by $x^3$ = 15. The compound of Formula I may be present in the phenolic composition in an amount from 15-75%, relative to the total mass of the phenolic composition, the compound of Formula II may be present in an amount from 15-75%, relative to the total mass of the phenolic composition, and the compound of Formula III may be present in an amount from 15-75%, relative to the total mass of the phenolic composition.

In some embodiments, the compound of Formula I is characterized by $x^1$=15, the compound of Formula II is characterized by $x^2$=14. The compound of Formula I may be present in the phenolic composition in an amount from 15-75%, relative to the total mass of the phenolic composition, and the compound of Formula II may be present in an amount from 15-75%, relative to the total mass of the phenolic composition.

In certain embodiments, the compound of Formula I is characterized by $x^1$=15, the compound of Formula II is characterized by $x^2$=14, and the compound of Formula III is characterized by $x^3$ = 16. The compound of Formula I may be present in the phenolic composition in an amount from 15-75%, relative to the total mass of the phenolic composition, the compound of Formula II may be present in an amount from 15-75%, relative to the total mass of the phenolic composition, and the compound of Formula III may be present in an amount from 15-75%, relative to the total mass of the phenolic composition.

In some embodiments, the compound of Formula I is characterized by $x^1$=16, the compound of Formula II is characterized by $x^2$=15 The compound of Formula I may be present in the phenolic composition in an amount from 15-75%, relative to the total mass of the phenolic composition, and the compound of Formula II may be present in an amount from 15-75%, relative to the total mass of the phenolic composition.

In certain embodiments, the compound of Formula I is characterized by $x^1$=16 the compound of Formula II is characterized by $x^2$=15, and the compound of Formula III is characterized by $x^3$ = 17. The compound of Formula I may be present in the phenolic composition in an amount from 15-75%, relative to the total mass of the phenolic composition, the compound of Formula II may be present in an amount from 15-75%, relative to the total mass of the phenolic composition, and the compound of Formula III may be present in an amount from 15-75%, relative to the total mass of the phenolic composition.

In some embodiments, the compound of Formula I is characterized by $x^1$=17, the compound of Formula II is characterized by $x^2$=16. The compound of Formula I may be present in the phenolic composition in an amount from 15-75%, relative to the total mass of the phenolic composition, and the compound of Formula II may be present in an amount from 15-75%, relative to the total mass of the phenolic composition.

In certain embodiments, the compound of Formula I is characterized by $x^1$=17, the compound of Formula II is characterized by $x^2$=16, and the compound of Formula III is characterized by $x^3$ = 18. The compound of Formula I may be present in the phenolic composition in an amount from 15-75%, relative to the total mass of the phenolic composition, the compound of Formula II may be present in an amount from 15-75%, relative to the total mass of the phenolic composition, and the compound of Formula III may be present in an amount from 15-75%, relative to the total mass of the phenolic composition.

In certain embodiments, the compound of Formula I is characterized by $x^1$=3, the compound of Formula II is characterized by $x^2$=4, the compound of Formula III is characterized by $x^3$=5, the compound of Formula IV is characterized by $x^4$=6, the compound of Formula V is characterized by $x^5$=7, the compound of Formula VI is characterized by $x^6$=8, the compound of Formula VII is characterized by $x^7$=9, and the compound of Formula VIII is characterized by $x^8$=10. These compounds may be present in the phenolic compositions as shown in the table below (expressed as weight percent relative to the total mass of the phenolic composition):

| I | II | III | IV | V | VI | VII | VIII |
|---|---|---|---|---|---|---|---|
| 25-75% | 25-75% | 0-50% | 0-50% | 0-50% | 0-50% | 0-50% | 0-50% |
| 25-75% | 25-75% | 25-75% | 0-25% | 0-25% | 0-25% | 0-25% | 0-25% |
| 0-50% | 25-75% | 25-75% | 0-50% | 0-50% | 0-50% | 0-50% | 0-50% |
| 0-25% | 25-75% | 25-75% | 25-75% | 0-25% | 0-25% | 0-25% | 0-25% |
| 0-50% | 0-50% | 25-75% | 25-75% | 0-50% | 0-50% | 0-50% | 0-50% |
| 0-25% | 0-25% | 25-75% | 25-75% | 25-75% | 0-25% | 0-25% | 0-25% |
| 0-50% | 0-50% | 0-50% | 25-75% | 25-75% | 0-50% | 0-50% | 0-50% |
| 0-25% | 0-25% | 0-25% | 25-75% | 25-75% | 25-75% | 0-25% | 0-25% |
| 0-50% | 0-50% | 0-50% | 0-50% | 25-75% | 25-75% | 0-50% | 0-50% |
| 0-25% | 0-25% | 0-25% | 0-25% | 25-75% | 25-75% | 25-75% | 0-25% |
| 0-50% | 0-50% | 0-50% | 0-50% | 0-50% | 25-75% | 25-75% | 0-50% |
| 0-25% | 0-25% | 0-25% | 0-25% | 0-25% | 25-75% | 25-75% | 25-75% |
| 15-40% | 15-40% | 15-35% | 5-15% | <10% | <5% | <3% | <3% |
| < 5% | 5-15% | 10-25% | 15-35% | 15-35% | 5-25% | 2-10% | <5% |
| <3% | <1% | <5% | 5-15% | 8-20% | 10-25% | 10-25% | 9-20% |
| 7-20% | 7-20% | 10-25% | 10-20% | 5-15% | 2-15% | 1-5% | <5% |
| 5-15% | 5-15% | 5-10% | < 5% | < 5% | 5-10% | 5-15% | 5-15% |
| 15-50% | 15-50% | 15-50% | 15-50% | 0-55% | 0-55% | 0-55% | 0-55% |
| 0-55% | 15-50% | 15-50% | 15-50% | 15-50% | 0-55% | 0-55% | 0-55% |
| 0-55% | 0-55% | 15-50% | 15-50% | 15-50% | 15-50% | 0-55% | 0-55% |
| 0-55% | 0-55% | 0-55% | 15-50% | 15-50% | 15-50% | 15-50% | 0-55% |
| 0-55% | 0-55% | 0-55% | 0-55% | 15-50% | 15-50% | 15-50% | 15-50% |
| 15-40% | 15-40% | 15-40% | 15-40% | 15-40% | 0-25% | 0-25% | 0-25% |
| 0-25% | 15-40% | 15-40% | 15-40% | 15-40% | 15-40% | 0-25% | 0-25% |
| 0-25% | 0-25% | 15-40% | 15-40% | 15-40% | 15-40% | 15-40% | 0-25% |
| 0-25% | 0-25% | 0-25% | 15-40% | 15-40% | 15-40% | 15-40% | 15-40% |

In certain embodiments, the compound of Formula I is characterized by $x^1$=6, the compound of Formula II is characterized by $x^2$=7, the compound of Formula III is characterized by $x^3$=8, the compound of Formula IV is characterized by $x^4$=9, the compound of Formula V is characterized by $x^5$=10, the compound of Formula VI is characterized by $x^6$=11, the compound of Formula VII is characterized by $x^7$=12, and the compound of Formula VIII is characterized by $x^8$=13. These compounds may be present in the phenolic compositions as shown in the table below (expressed as weight percent relative to the total mass of the phenolic composition):

| I | II | III | IV | V | VI | VII | VIII |
|---|---|---|---|---|---|---|---|
| 25-75% | 25-75% | 0-50% | 0-50% | 0-50% | 0-50% | 0-50% | 0-50% |
| 25-75% | 25-75% | 25-75% | 0-25% | 0-25% | 0-25% | 0-25% | 0-25% |
| 0-50% | 25-75% | 25-75% | 0-50% | 0-50% | 0-50% | 0-50% | 0-50% |
| 0-25% | 25-75% | 25-75% | 25-75% | 0-25% | 0-25% | 0-25% | 0-25% |
| 0-50% | 0-50% | 25-75% | 25-75% | 0-50% | 0-50% | 0-50% | 0-50% |
| 0-25% | 0-25% | 25-75% | 25-75% | 25-75% | 0-25% | 0-25% | 0-25% |
| 0-50% | 0-50% | 0-50% | 25-75% | 25-75% | 0-50% | 0-50% | 0-50% |

(continued)

| I | II | III | IV | V | VI | VII | VIII |
|---|---|---|---|---|---|---|---|
| 0-25% | 0-25% | 0-25% | 25-75% | 25-75% | 25-75% | 0-25% | 0-25% |
| 0-50% | 0-50% | 0-50% | 0-50% | 25-75% | 25-75% | 0-50% | 0-50% |
| 0-25% | 0-25% | 0-25% | 0-25% | 25-75% | 25-75% | 25-75% | 0-25% |
| 0-50% | 0-50% | 0-50% | 0-50% | 0-50% | 25-75% | 25-75% | 0-50% |
| 0-25% | 0-25% | 0-25% | 0-25% | 0-25% | 25-75% | 25-75% | 25-75% |
| 15-40% | 15-40% | 15-35% | 5-15% | <10% | <5% | <3% | <3% |
| < 5% | 5-15% | 10-25% | 15-35% | 15-35% | 5-25% | 2-10% | <5% |
| <3% | <1% | <5% | 5-15% | 8-20% | 10-25% | 10-25% | 9-20% |
| 7-20% | 7-20% | 10-25% | 10-20% | 5-15% | 2-15% | 1-5% | <5% |
| 5-15% | 5-15% | 5-10% | < 5% | < 5% | 5-10% | 5-15% | 5-15% |
| 15-50% | 15-50% | 15-50% | 15-50% | 0-55% | 0-55% | 0-55% | 0-55% |
| 0-55% | 15-50% | 15-50% | 15-50% | 15-50% | 0-55% | 0-55% | 0-55% |
| 0-55% | 0-55% | 15-50% | 15-50% | 15-50% | 15-50% | 0-55% | 0-55% |
| 0-55% | 0-55% | 0-55% | 15-50% | 15-50% | 15-50% | 15-50% | 0-55% |
| 0-55% | 0-55% | 0-55% | 0-55% | 15-50% | 15-50% | 15-50% | 15-50% |
| 15-40% | 15-40% | 15-40% | 15-40% | 15-40% | 0-25% | 0-25% | 0-25% |
| 0-25% | 15-40% | 15-40% | 15-40% | 15-40% | 15-40% | 0-25% | 0-25% |
| 0-25% | 0-25% | 15-40% | 15-40% | 15-40% | 15-40% | 15-40% | 0-25% |
| 0-25% | 0-25% | 0-25% | 15-40% | 15-40% | 15-40% | 15-40% | 15-40% |

In certain embodiments, the compound of Formula I is characterized by $x^1$=10, the compound of Formula II is characterized by $x^2$=11, the compound of Formula III is characterized by $x^3$=12, the compound of Formula IV is characterized by $x^1$=13, the compound of Formula V is characterized by $x^5$=14, the compound of Formula VI is characterized by $x^6$=15, the compound of Formula VII is characterized by $x^7$=16, and the compound of Formula VIII is characterized by $x^8$=17. These compounds may be present in the phenolic compositions as shown in the table below (expressed as weight percent relative to the total mass of the phenolic composition):

| I | II | III | IV | V | VI | VII | VIII |
|---|---|---|---|---|---|---|---|
| 25-75% | 25-75% | 0-50% | 0-50% | 0-50% | 0-50% | 0-50% | 0-50% |
| 25-75% | 25-75% | 25-75% | 0-25% | 0-25% | 0-25% | 0-25% | 0-25% |
| 0-50% | 25-75% | 25-75% | 0-50% | 0-50% | 0-50% | 0-50% | 0-50% |
| 0-25% | 25-75% | 25-75% | 25-75% | 0-25% | 0-25% | 0-25% | 0-25% |
| 0-50% | 0-50% | 25-75% | 25-75% | 0-50% | 0-50% | 0-50% | 0-50% |
| 0-25% | 0-25% | 25-75% | 25-75% | 25-75% | 0-25% | 0-25% | 0-25% |
| 0-50% | 0-50% | 0-50% | 25-75% | 25-75% | 0-50% | 0-50% | 0-50% |
| 0-25% | 0-25% | 0-25% | 25-75% | 25-75% | 25-75% | 0-25% | 0-25% |
| 0-50% | 0-50% | 0-50% | 0-50% | 25-75% | 25-75% | 0-50% | 0-50% |
| 0-25% | 0-25% | 0-25% | 0-25% | 25-75% | 25-75% | 25-75% | 0-25% |
| 0-50% | 0-50% | 0-50% | 0-50% | 0-50% | 25-75% | 25-75% | 0-50% |
| 0-25% | 0-25% | 0-25% | 0-25% | 0-25% | 25-75% | 25-75% | 25-75% |
| 15-40% | 15-40% | 15-35% | 5-15% | <10% | <5% | <3% | <3% |

(continued)

| I | II | III | IV | V | VI | VII | VIII |
|---|---|---|---|---|---|---|---|
| < 5% | 5-15% | 10-25% | 15-35% | 15-35% | 5-25% | 2-10% | <5% |
| <3% | <1% | <5% | 5-15% | 8-20% | 10-25% | 10-25% | 9-20% |
| 7-20% | 7-20% | 10-25% | 10-20% | 5-15% | 2-15% | 1-5% | <5% |
| 5-15% | 5-15% | 5-10% | < 5% | < 5% | 5-10% | 5-15% | 5-15% |
| 15-50% | 15-50% | 15-50% | 15-50% | 0-55% | 0-55% | 0-55% | 0-55% |
| 0-55% | 15-50% | 15-50% | 15-50% | 15-50% | 0-55% | 0-55% | 0-55% |
| 0-55% | 0-55% | 15-50% | 15-50% | 15-50% | 15-50% | 0-55% | 0-55% |
| 0-55% | 0-55% | 0-55% | 15-50% | 15-50% | 15-50% | 15-50% | 0-55% |
| 0-55% | 0-55% | 0-55% | 0-55% | 15-50% | 15-50% | 15-50% | 15-50% |
| 15-40% | 15-40% | 15-40% | 15-40% | 15-40% | 0-25% | 0-25% | 0-25% |
| 0-25% | 15-40% | 15-40% | 15-40% | 15-40% | 15-40% | 0-25% | 0-25% |
| 0-25% | 0-25% | 15-40% | 15-40% | 15-40% | 15-40% | 15-40% | 0-25% |
| 0-25% | 0-25% | 0-25% | 15-40% | 15-40% | 15-40% | 15-40% | 15-40% |

Exemplary phenolic compounds that may be used in the phenolic compositions include:

| Cpd. | $R^1$ | $R^2$ | L | $R^3$ | X |
|---|---|---|---|---|---|
| 2-A | $C(CH3)_3$ | $CH_3$ | $-CH_2CH_2-$ | H | 2 |
| 2-B | $C(CH3)_3$ | $CH_2CH_3$ | $-CH_2CH_2-$ | H | 2 |
| 2-C | $C(CH3)_3$ | $CH_3$ | $-CH_2CH_2-$ | $CH_3$ | 2 |
| 2-D | $C(CH3)_3$ | $CH_2CH_3$ | $-CH_2CH_2-$ | $CH_3$ | 2 |
| 3-A | $C(CH3)_3$ | $CH_3$ | $-CH_2CH_2-$ | H | 3 |
| 3-B | $C(CH3)_3$ | $CH_2CH_3$ | $-CH_2CH_2-$ | H | 3 |
| 3-C | $C(CH3)_3$ | $CH_3$ | $-CH_2CH_2-$ | $CH_3$ | 3 |
| 3-D | $C(CH3)_3$ | $CH_2CH_3$ | $-CH_2CH_2-$ | $CH_3$ | 3 |
| 4-A | $C(CH3)_3$ | $CH_3$ | $-CH_2CH_2-$ | H | 4 |
| 4-B | $C(CH3)_3$ | $CH_2CH_3$ | $-CH_2CH_2-$ | H | 4 |
| 4-C | $C(CH3)_3$ | $CH_3$ | $-CH_2CH_2-$ | $CH_3$ | 4 |
| 4-D | $C(CH3)_3$ | $CH_2CH_3$ | $-CH_2CH_2-$ | $CH_3$ | 4 |
| 5-A | $C(CH3)_3$ | $CH_3$ | $-CH_2CH_2-$ | H | 5 |
| 5-B | $C(CH3)_3$ | $CH_2CH_3$ | $-CH_2CH_2-$ | H | 5 |
| 5-C | $C(CH3)_3$ | $CH_3$ | $-CH_2CH_2-$ | $CH_3$ | 5 |
| 5-D | $C(CH3)_3$ | $CH_2CH_3$ | $-CH_2CH_2-$ | $CH_3$ | 5 |
| 6-A | $C(CH3)_3$ | $CH_3$ | $-CH_2CH_2-$ | H | 6 |
| 6-B | $C(CH3)_3$ | $CH_2CH_3$ | $-CH_2CH_2-$ | H | 6 |
| 6-C | $C(CH3)_3$ | $CH_3$ | $-CH_2CH_2-$ | $CH_3$ | 6 |
| 6-D | $C(CH3)_3$ | $CH_2CH_3$ | $-CH_2CH_2-$ | $CH_3$ | 6 |
| 7-A | $C(CH3)_3$ | $CH_3$ | $-CH_2CH_2-$ | H | 7 |
| 7-B | $C(CH3)_3$ | $CH_2CH_3$ | $-CH_2CH_2-$ | H | 7 |
| 7-C | $C(CH3)_3$ | $CH_3$ | $-CH_2CH_2-$ | $CH_3$ | 7 |

(continued)

| Cpd. | $R^1$ | $R^2$ | L | $R^3$ | X |
|---|---|---|---|---|---|
| 7-D | $C(CH3)_3$ | $CH_2CH_3$ | $-CH_2CH_2-$ | $CH_3$ | 7 |
| 8-A | $C(CH3)_3$ | $CH_3$ | $-CH_2CH_2-$ | H | 8 |
| 8-B | $C(CH3)_3$ | $CH_2CH_3$ | $-CH_2CH_2-$ | H | 8 |
| 8-C | $C(CH3)_3$ | $CH_3$ | $-CH_2CH_2-$ | $CH_3$ | 8 |
| 8-D | $C(CH3)_3$ | $CH_2CH_3$ | $-CH_2CH_2-$ | $CH_3$ | 8 |
| 9-A | $C(CH3)_3$ | $CH_3$ | $-CH_2CH_2-$ | H | 9 |
| 9-B | $C(CH3)_3$ | $CH_2CH_3$ | $-CH_2CH_2-$ | H | 9 |
| 9-C | $C(CH3)_3$ | $CH_3$ | $-CH_2CH_2-$ | $CH_3$ | 9 |
| 9-D | $C(CH3)_3$ | $CH_2CH_3$ | $-CH_2CH_2-$ | $CH_3$ | 9 |
| 10-A | $C(CH3)_3$ | $CH_3$ | $-CH_2CH_2-$ | H | 10 |
| 10-B | $C(CH3)_3$ | $CH_2CH_3$ | $-CH_2CH_2-$ | H | 10 |
| 10-C | $C(CH3)_3$ | $CH_3$ | $-CH_2CH_2-$ | $CH_3$ | 10 |
| 10-D | $C(CH3)_3$ | $CH_2CH_3$ | $-CH_2CH_2-$ | $CH_3$ | 10 |
| 11-A | $C(CH3)_3$ | $CH_3$ | $-CH_2CH_2-$ | H | 11 |
| 11-B | $C(CH3)_3$ | $CH_2CH_3$ | $-CH_2CH_2-$ | H | 11 |
| 11-C | $C(CH3)_3$ | $CH_3$ | $-CH_2CH_2-$ | $CH_3$ | 11 |
| 11-D | $C(CH3)_3$ | $CH_2CH_3$ | $-CH_2CH_2-$ | $CH_3$ | 11 |
| 12-A | $C(CH3)_3$ | $CH_3$ | $-CH_2CH_2-$ | H | 12 |
| 12-B | $C(CH3)_3$ | $CH_2CH_3$ | $-CH_2CH_2-$ | H | 12 |
| 12-C | $C(CH3)_3$ | $CH_3$ | $-CH_2CH_2-$ | $CH_3$ | 12 |
| 12-D | $C(CH3)_3$ | $CH_2CH_3$ | $-CH_2CH_2-$ | $CH_3$ | 12 |
| 13-A | $C(CH3)_3$ | $CH_3$ | $-CH_2CH_2-$ | H | 13 |
| 13-B | $C(CH3)_3$ | $CH_2CH_3$ | $-CH_2CH_2-$ | H | 13 |
| 13-C | $C(CH3)_3$ | $CH_3$ | $-CH_2CH_2-$ | $CH_3$ | 13 |
| 13-D | $C(CH3)_3$ | $CH_2CH_3$ | $-CH_2CH_2-$ | $CH_3$ | 13 |
| 14-A | $C(CH3)_3$ | $CH_3$ | $-CH_2CH_2-$ | H | 14 |
| 14-B | $C(CH3)_3$ | $CH_2CH_3$ | $-CH_2CH_2-$ | H | 14 |
| 14-C | $C(CH3)_3$ | $CH_3$ | $-CH_2CH_2-$ | $CH_3$ | 14 |
| 14-D | $C(CH3)_3$ | $CH_2CH_3$ | $-CH_2CH_2-$ | $CH_3$ | 14 |
| 15-A | $C(CH3)_3$ | $CH_3$ | $-CH_2CH_2-$ | H | 15 |
| 15-B | $C(CH3)_3$ | $CH_2CH_3$ | $-CH_2CH_2-$ | H | 15 |
| 15-C | $C(CH3)_3$ | $CH_3$ | $-CH_2CH_2-$ | $CH_3$ | 15 |
| 15-D | $C(CH3)_3$ | $CH_2CH_3$ | $-CH_2CH_2-$ | $CH_3$ | 15 |

Impurities may also result from manufacturing, processing, transportation, storage and use of the inventive materials.

Such impurities can be present from by-products of the reaction process to make the inventive materials, such as loss of an $R^1$ or $R^2$ group, hydrolysis of the ester linkage to a carboxylic acid, single transesterification with the PEG chain resulting in a pegylated ester wherein the PEG terminates in an -OH group. Additional impurities of the mono-ester of PEG impurity can result in autoxidized products, wherein the -OH may form -CHO or $-CO_2H$ groups. These molecules could be considered as impurities, and their presence will not negate the present invention so long as the majority (and preferably the substantial majority) of the molecules of the composition are as described. In any event, compositions exhibiting this

type of variability are to be construed as encompassed by the present invention and the description that a material is "represented by" the formula shown.

Impurities in the phenolic compositions can also result from autoxidation of the product or from trapping of radicals. These impurities are expected to be a part of the product, especially during usage in the detergent or in application. Examples of expected impurities from oxidation can be found in Zhang et al., J Material Sci Eng. 6: 393.

The phenolic compositions disclosed herein may be formulated into a wide variety of laundry care compositions. Suitable laundry care compositions include powdered laundry detergents, detergent tablets and bars, wash additives, laundry detergent liquids including light duty liquids, heavy duty liquids, concentrated liquid detergents, non or low aqueous laundry liquids, unit dose sachets, soap bars, laundry beads, dryer sheets, dissolvable sheets, and combinations thereof. The phenolic composition may be present in an amount from 0.0001 wt.% - 1.0 wt.%, from 0.0001 wt.% - 0.5 wt.%, from 0.0001 wt.% - 0.1 wt.%, from 0.0001 wt.% - 0.01 wt.%, from 0.0001 wt.% - 0.001 wt.%, from 0.001 wt.% - 1.0 wt.%, from 0.001 wt.% - 1.0 wt.%, from 0.01 wt.% - 1.0 wt.%, from 0.1 wt.% - 1.0 wt.%, or from 0.5 wt.% - 1.0 wt.%, relative to the total weight of the laundry care composition.

The laundry care composition may be in the form of a liquid composition. The liquid composition may comprise from about 0% to about 99%, or from about 30% to about 90%, or from about 50% to about 80%, by weight of the composition, of water. The liquid composition may include non-aqueous liquid detergents.

The laundry care composition may be in the form of a solid composition. The solid composition may comprise from about 20% to about 98%, by weight of the composition, of a water-soluble carrier for forming solid compositions. In a non-limiting but preferred example, the water-soluble carrier for forming solid compositions may be polyethylene glycol. The polyethylene glycol carrier may have a weight average molecular weight of from about 2000 to about 20,000 Daltons, preferably from about 5000 to about 15,000 Daltons, more preferably from about 6000 to about 12,000 Daltons. The solid composition may comprise less than about 20%, preferably less than about 15%, more preferably less than about 5%, even more preferably less than about 1%, by weight of the composition, of water. In a preferred example, the laundry care composition is in the form of granules or particles. The granules and particles may have a shape selected from the group consisting of spherical, hemispherical, compressed hemispherical, lentil shaped, oblong, and mixtures thereof. One skilled in the art may recognize that these shapes are non-limiting and that the granules and particles may have any other shape known in the art for such granules and particles. The granules may have a maximum dimension (i.e. , length, width, height, diameter) of from about 0.1 mm to about 2 mm and a minimum dimension (i.e., length, width, height, diameter) of from about 0.05 mm to about 1.5 mm. The particles may have a maximum dimension (i.e., length, width, height, diameter) of from about 2 mm to about 10 mm and a minimum dimension (i.e., length, width, height, diameter) of from about 1.5 mm to about 4 mm.

The laundry care composition may be free-flowing. Such free-flowing laundry care compositions may be packaged within a container such that a consumer may open the container and simply dose the amount of laundry care composition desired. The container may be any container known in the art suitable for containing laundry care compositions. For example, the container may have a volume of from about 50 $cm^3$ to about 1500 $cm^3$. The container may be of any suitable size and shape for placement on a grocery store shelf, for placement within a consumer's home, or for use within a commercial setting, such as a laundromat.

It is also contemplated that the laundry care composition may be incorporated into a unitized dose article, such as, for example, a single-compartment pouch, a multi-compartment pouch, a dissolvable sheet, a fibrous article, a tablet, a bar, or a mixture thereof. Such pouches typically include a water-soluble film, such as a polyvinyl alcohol water-soluble film, that at least partially encapsulates the laundry care composition. Suitable films include those commercially available from MonoSol, LLC, Indiana, United States. A multi-compartment pouch may comprise at least two, at least three, or at least four compartments. A multi-compartment pouch may include compartments that are side-by-side and/or superposed. The laundry care composition contained in the pouch or compartments thereof may be of liquid form, of solid form, or combinations thereof.

Laundry care compositions encapsulated within pouches may have relatively low amounts of water, for example less than about 20%, or less than about 15%, or less than about 12%, or less than about 10%, or less than about 8%, by weight of the laundry care composition, of water.

The laundry care composition includes one or more surfactants, for instance in an amount about 0.1% to about 80%, by weight of the laundry care composition, of a surfactant. The surfactant may be selected from the group consisting of nonionic surfactants, anionic surfactants, amphoteric surfactants, zwitterionic surfactants, cationic surfactants, and mixtures thereof. Anionic and nonionic surfactants are typically employed if the laundry care composition is a laundry cleaning composition or detergent. Cationic surfactants are typically employed if the laundry care composition is a fabric softening composition. Surfactants may provide soil removal and assist in dispersing the antioxidant, while not negatively impacting the deposition of the antioxidant onto the fabric.

Suitable nonionic surfactants may include, but are not limited to, alkoxylated fatty alcohols (e.g., ethoxylated fatty alcohols); alkoxylated alkyl phenols; alkyl phenol condensates; mid-chain branched alcohols; mid-chain branched alkyl alkoxylates; alkylpolysaccharides; polyhydroxy fatty acid amides; ether capped poly(oxyalkylated) alcohol surfactants;

and mixtures thereof. The alkoxylate units may be ethyleneoxy units, propyleneoxy units, or mixtures thereof. The nonionic surfactant may be linear, branched (e.g., mid-chain branched), or a combination thereof. Examples of suitable nonionic surfactants may include those commercially available under the tradename PLURONIC® from BASF, Ludwigshafen, Germany, those commercially available under the tradename NEODOL® nonionic from Shell, The Hague, The Netherlands, and those commercially available under the tradename SURFONIC® from Huntsman Corporation, The Woodlands, Tex., United States.

Suitable anionic surfactants may include, but are not limited to sulfate detersive surfactants (e.g., alkoxylated and/or non-alkoxylated alkyl sulfate materials); and/or sulfonic detersive surfactants (e.g., alkyl benzene sulfonates). The anionic surfactant may be linear, branched, or combinations thereof. Preferred anionic surfactants may include, but are not limited to, linear alkyl benzene sulfonate (LAS), alkyl ethoxylated sulfate (AES), alkyl sulfates (AS), and mixtures thereof. Other suitable anionic surfactants may include branched modified alkyl benzene sulfonates (MLAS), methyl ester sulfonates (MES), and/or alkyl ethoxylated carboxylates (AEC). The anionic surfactants may be present in acid form, salt form, or mixtures thereof. The anionic surfactant may be neutralized, in part or in whole, for example, by an alkali metal (e.g., sodium) or an amine (e.g., monoethanolamine). The anionic surfactant may be pre-neutralized, preferably with an alkali metal, an alkali earth metal, an amine such as an ethanolamine, or mixtures thereof.

Suitable amphoteric surfactants may include any conventional amphoteric surfactant known to one skilled in the art, such as amine oxides. Preferred amine oxides may include alkyl dimethyl amine oxide or alkyl amido propyl dimethyl amine oxide, more preferably alkyl dimethyl amine oxide and even more preferably coco dimethyl amino oxide. The amine oxide may have a linear or mid-branched alkyl moiety. Typical linear amine oxides may include water-soluble amine oxides containing one $C_{8-18}$alkyl moiety and two moieties selected from the group consisting of $C_{1-3}$alkyl groups, $C_{1-3}$hydroxyalkyl groups, and mixtures thereof. Preferably, the amine oxide is characterized by the formula $R^4$-N($R^5$)($R^6$)O wherein $R^4$ is a C8-18 alkyl and $R^5$ and $R^6$ are selected from the group consisting of methyl, ethyl, propyl, isopropyl, 2-hydroxyethyl, 2-hydroxypropyl and 3-hydroxypropyl. The amine oxide surfactant may include linear $C_{10}$-$C_{18}$alkyl dimethyl amine oxides and linear $C_8$-$C_{12}$ alkoxy ethyl dihydroxy ethyl amine oxides.

Suitable zwitterionic surfactants may include any conventional zwitterionic surfactant known to one skilled in the art, such as betaines, particularly alkyl betaine, alkylamidobetaine, amidazoliniumbetaine, sulfobetaine, hydroxybetaines, and phosphobetaines. Examples of suitable betaines may include alkyl dimethyl betaine and cocodimethyl amidopropyl betaine, N-alkyl-N,N-dimethylammino-1-propane sulfonate where the alkyl group can be $C_8$ to $C_{18}$, or from $C_8$ to $C_{14}$. Suitable cationic surfactants may include, but are not limited to, alkyl pyridinium compounds, alkyl quaternary ammonium compounds, alkyl quaternary phosphonium compounds, alkyl ternary sulphonium compounds, and mixtures thereof. Preferred cationic surfactants are quaternary ammonium compounds having the general formula:
($R^7$)($R^8$)($R^9$)($R^{10}$-)$N^+$ $X^-$ wherein, $R^7$ is a linear or branched, substituted or unsubstituted $C_{6-18}$ alkyl or alkenyl moiety, $R^8$ and $R^9$ are independently selected from methyl or ethyl moieties, $R^{10}$ is a hydroxyl, hydroxymethyl or a hydroxyethyl moiety, X is an anion which provides charge neutrality, preferred anions include: halides, preferably chloride; sulphate; and sulphonate. For the purposes of the present invention, cationic surfactants include those which can deliver fabric care benefits. Non-limiting examples of useful cationic surfactants include: fatty amines, imidazoline quat materials and quaternary ammonium surfactants, preferably N, N-bis(stearoyl-oxy-ethyl) N,N-dimethyl ammonium chloride, N,N-bis(tallowoyl-oxy-ethyl) N,N-dimethyl ammonium chloride, N,N-bis(stearoyl-oxy-ethyl) N-(2 hydroxyethyl) N-methyl ammonium methylsulfate; 1,2-di(stearoyl-oxy) 3-trimethyl ammoniumpropane chloride; dialkylenedimethylammonium salts such as dicanoladimethylammonium chloride, di(hard)tallowdimethylammonium chloride dicanoladimethylammonium methylsulfate; 1-methyl-1-stearoylamidoethyl-2-stearoylimidazolinium methylsulfate; 1-tallowylamidoethyl-2-tallowylimidazoline; N,N"-dialkyldiethylenetriamine; the reaction product of N-(2-hydroxyethyl)-1,2-ethylenediamine or N-(2-hydroxyisopropyl)-1,2-ethylenediamine with glycolic acid, esterified with fatty acid, where the fatty acid is (hydrogenated) tallow fatty acid, palm fatty acid, hydrogenated palm fatty acid, oleic acid, rapeseed fatty acid, hydrogenated rapeseed fatty acid, and a mixture of the above.

The detergent compositions of the present invention may also include any number of additional optional ingredients. These include conventional laundry detergent composition components such as non-tinting dyes, detersive builders, enzymes, enzyme stabilizers (such as propylene glycol, boric acid and/or borax), suds suppressors, soil suspending agents, hueing agents, soil release agents, other fabric care benefit agents, pH adjusting agents, chelating agents, smectite clays, solvents, hydrotropes and phase stabilizers, structuring agents, dye transfer inhibiting agents, opacifying agents, optical brighteners, perfumes and coloring agents. The various optional detergent composition ingredients, if present in the compositions herein, should be utilized at concentrations conventionally employed to bring about their desired contribution to the composition or the laundering operation. Frequently, the total amount of such optional detergent composition ingredients can range from about 0.01% to about 50%, more preferably from about 0.1% to about 30%, by weight of the composition.

Builders - The compositions of the present invention can comprise one or more detergent builders or builder systems. When present, the compositions will typically comprise at least about 1% builder, or from about 5% or 10% to about 80%, 50%, or even 30% by weight, of said builder. Builders include, but are not limited to, the alkali metal, ammonium

and alkanolammonium salts of polyphosphates, alkali metal silicates, alkaline earth and alkali metal carbonates, aluminosilicate builders polycarboxylate compounds. ether hydroxypolycarboxylates, copolymers of maleic anhydride with ethylene or vinyl methyl ether, 1,3,5-trihydroxybenzene-2,4,6-trisulphonic acid, and carboxymethyl-oxysuccinic acid, the various alkali metal, ammonium and substituted ammonium salts of polyacetic acids such as ethylenediamine tetraacetic acid and nitrilotriacetic acid, as well as polycarboxylates such as mellitic acid, succinic acid, oxydisuccinic acid, polymaleic acid, benzene 1,3,5-tricarboxylic acid, carboxymethyloxysuccinic acid, and soluble salts thereof.

Chelating Agents - The compositions herein may also optionally contain one or more copper, iron and/or manganese chelating agents. If utilized, chelating agents will generally comprise from about 0.1% by weight of the compositions herein to about 15%, or even from about 3.0% to about 15% by weight of the compositions herein.

Hueing Agents - The compositions of the present invention may also include one or more hueing agents. Suitable hueing agents consist of blue or violet azo colorants, triarylmethane dyes or various leuco dyes. Various non-limiting examples of hueing agents are disclosed in U.S. Patent Nos. 10,731,112 and 10,876,079. When present in the compositions herein, the hueing dye is present at levels from about 0.0001%, from about 0.01%, from about 0.05% by weight of the cleaning compositions to about 1%, or even about 0.1% by weight of the cleaning compositions.

Dye Transfer Inhibiting Agents - The compositions of the present invention may also include one or more dye transfer inhibiting agents. Suitable polymeric dye transfer inhibiting agents include, but are not limited to, polyvinylpyrrolidone polymers, polyamine N-oxide polymers, copolymers of N-vinylpyrrolidone and N-vinylimidazole, polyvinyloxazolidones and polyvinylimidazoles or mixtures thereof. When present in the compositions herein, the dye transfer inhibiting agents are present at levels from about 0.0001%, from about 0.01%, from about 0.05% by weight of the cleaning compositions to about 10%, about 2%, or even about 1% by weight of the cleaning compositions.

Dispersants - The compositions of the present invention can also contain dispersants. Suitable water-soluble organic materials are the homo- or co-polymeric acids or their salts, in which the polycarboxylic acid may comprise at least two carboxyl radicals separated from each other by not more than two carbon atoms.

Enzymes - The compositions can comprise one or more detergent enzymes which provide cleaning performance and/or fabric care benefits. Examples of suitable enzymes include, but are not limited to, hemicellulases, peroxidases, proteases, cellulases, xylanases, lipases, phospholipases, esterases, cutinases, pectinases, keratanases, reductases, oxidases, phenoloxidases, lipoxygenases, ligninases, pullulanases, tannases, pentosanases, malanases, β-glucanases, arabinosidases, hyaluronidase, chondroitinase, laccase, and amylases, or mixtures thereof. A typical combination is a cocktail of conventional applicable enzymes like protease, lipase, cutinase and/or cellulase in conjunction with amylase.

Enzyme Stabilizers - Enzymes for use in compositions, for example, detergents can be stabilized by various techniques. The enzymes employed herein can be stabilized by the presence of water-soluble sources of calcium and/or magnesium ions in the finished compositions that provide such ions to the enzymes.

Detergent compositions may also contain bleaching agents. Suitable bleaching agents include, for example, hydrogen peroxide sources, such as those described in detail in the herein incorporated Kirk Othmer's Encyclopedia of Chemical Technology, 4th Ed (1992, John Wiley & Sons), Vol. 4, pp. 271-300 "Bleaching Agents (Survey)." These hydrogen peroxide sources include the various forms of sodium perborate and sodium percarbonate, including various coated and modified forms of these compounds.

The preferred source of hydrogen peroxide used herein can be any convenient source, including hydrogen peroxide itself. For example, perborate, e.g., sodium perborate (any hydrate but preferably the mono- or tetra-hydrate), sodium carbonate peroxyhydrate or equivalent percarbonate salts, sodium pyrophosphate peroxyhydrate, urea peroxyhydrate, or sodium peroxide can be used herein. Also useful are sources of available oxygen such as persulfate bleach (e.g., OXONE, manufactured by DuPont). Sodium perborate monohydrate and sodium percarbonate are particularly preferred. Mixtures of any convenient hydrogen peroxide sources can also be used.

Bleach Activators - Preferably, the peroxygen bleach component in the composition is formulated with an activator (peracid precursor). The activator is present at levels of from about 0.01%, preferably from about 0.5%, more preferably from about 1% to about 15%, preferably to about 10%, more preferably to about 8%, by weight of the composition. A bleach activator as used herein is any compound which, when used in conjunction with a hydrogen peroxide, source leads to the in situ production of the peracid corresponding to the bleach activator. Various non-limiting examples of activators are disclosed in U.S. Patent Nos. 5,576,282; 4,915,854 and 4,412,934. See also U.S. Patent No. 4,634,551 for other typical bleaches and activators useful herein.

Preferred activators are selected from the group consisting of tetraacetyl ethylene diamine (TAED), benzoylcaprolactam (BzCL), 4-nitrobenzoylcaprolactam, 3-chlorobenzoylcaprolactam, benzoyloxybenzenesulphonate (BOBS), nonanoyloxybenzenesulphonate (NOBS), phenyl benzoate (PhBz), decanoyloxybenzenesulphonate ($C_{10}$-OBS), benzoylvalerolactam (BZVL), octanoyloxybenzenesulphonate ($C_8$-OBS), perhydrolyzable esters and mixtures thereof, most preferably benzoylcaprolactam and benzoylvalerolactam. Particularly preferred bleach activators in the pH range from about 8 to about 11 are those selected having an OBS or VL leaving group.

Preferred hydrophobic bleach activators include, but are not limited to, nonanoyloxybenzenesulphonate (NOBS); 4-[N-(nonanoyl) amino hexanoyloxy]-benzene sulfonate sodium salt (NACA-OBS), an example of which is

described in U.S. Patent No. 5,523,434; dodecanoyloxybenzenesulphonate (LOBS or $C_{12}$-OBS); 10-undecenoyloxybenzenesulfonate (UDOBS or $C_{11}$-OBS with unsaturation in the 10 position); and decanoyloxybenzoic acid (DOBA).

Preferred bleach activators are those described in U.S. Patent No. 5,998,350 to Burns et al.; U.S. Patent No. 5,698,504 to Christie et al.; U.S. Patent No. 5,695,679 to Christie et al.; U.S. Patent No. 5,686,401 to Willey et al.; U.S. Patent No. 5,686,014 to Hartshorn et al.; U.S. Patent No. 5,405,412 to Willey et al.; U.S. Patent No. 5,405,413 to Willey et al.; U.S. Patent No. 5,130,045 to Mitchel et al.; and U.S. Patent No. 4,412,934 to Chung et al., and copending Patent Application Serial No. 08/064,564.

Metal-Containing Bleach Catalysts - The compositions and methods of the present invention can also optionally include metal-containing bleach catalysts, preferably manganese and cobalt-containing bleach catalysts.

One type of metal-containing bleach catalyst is a catalyst system comprising a transition metal cation of defined bleach catalytic activity (such as copper, iron, titanium, ruthenium tungsten, molybdenum, or manganese cations), an auxiliary metal cation having little or no bleach catalytic activity (such as zinc or aluminum cations), and a sequestrate having defined stability constants for the catalytic and auxiliary metal cations, particularly ethylenediaminetetraacetic acid, ethylenediaminetetra (methylenephosphonic acid) and water-soluble salts thereof. Such catalysts are disclosed in U.S. Patent No. 4,430,243 to Bragg.

Bleach Boosting Compounds - The compositions herein may comprise one or more bleach boosting compounds. Bleach boosting compounds provide increased bleaching effectiveness in lower temperature applications. The bleach boosters act in conjunction with conventional peroxygen bleaching sources to provide increased bleaching effectiveness. This is normally accomplished through in situ formation of an active oxygen transfer agent such as a dioxirane, an oxaziridine, or an oxaziridinium. Alternatively, preformed dioxiranes, oxaziridines and oxaziridiniums may be used.

Among suitable bleach boosting compounds for use in accordance with the present invention are cationic imines, zwitterionic imines, anionic imines and/or polyionic imines having a net charge of from about +3 to about -3, and mixtures thereof. These imine bleach boosting compounds of the present invention include those of the general structure:

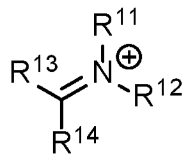

where $R^{11}$, $R^{12}$, $R^{13}$, and $R^{14}$ are each independently hydrogen or an unsubstituted or substituted radical selected from the group consisting of phenyl, aryl, heterocyclic ring, alkyl and cycloalkyl radicals.

Among preferred bleach boosting compounds are zwitterionic bleach boosters, which are described in U.S. Patent Nos. 5,576,282 and 5,718,614. Other bleach boosting compounds include cationic bleach boosters described in U.S. Patent Nos. 5,360,569; 5,442,066; 5,478,357; 5,370,826; 5,482,515; 5,550,256; and WO 95/13351, WO 95/13352, and WO 95/13353.

Peroxygen sources are well-known in the art and the peroxygen source employed in the present invention may comprise any of these well known sources, including peroxygen compounds as well as compounds, which under consumer use conditions, provide an effective amount of peroxygen in situ. The peroxygen source may include a hydrogen peroxide source, the in situ formation of a peracid anion through the reaction of a hydrogen peroxide source and a bleach activator, preformed peracid compounds or mixtures of suitable peroxygen sources. Of course, one of ordinary skill in the art will recognize that other sources of peroxygen may be employed without departing from the scope of the invention. The bleach boosting compounds, when present, are preferably employed in conjunction with a peroxygen source in the bleaching systems of the present invention.

Photobleaches - Suitable photobleaches for use in the treating compositions of the present invention include, but are not limited to, the photobleaches described in U.S. Patent Nos. 4,217,105 and 5,916,481.

Enzyme Bleaching - Enzymatic systems may be used as bleaching agents. The hydrogen peroxide may also be present by adding an enzymatic system (i.e. an enzyme and a substrate therefore) which is capable of generating hydrogen peroxide at the beginning or during the washing and/or rinsing process. Such enzymatic systems are disclosed in EP Patent Application 91202655.6 filed October 9, 1991.

The liquid detergent compositions are in the form of an aqueous solution or uniform dispersion or suspension of surfactant, phenolic composition, and certain optional other ingredients, some of which may normally be in solid form, that have been combined with the normally liquid components of the composition, such as the liquid alcohol ethoxylate nonionic, the aqueous liquid carrier, and any other normally liquid optional ingredients. Such a solution, dispersion or suspension will be acceptably phase stable and will typically have a viscosity which ranges from about 100 to 600 cps, more preferably from about 150 to 400 cps. For purposes of this invention, viscosity is measured with a Brookfield LVDV-II+ viscometer apparatus using a #21 spindle.

The liquid detergent compositions herein can be prepared by combining the components thereof in any

convenient order and by mixing, e.g., agitating, the resulting component combination to form a phase stable liquid detergent composition. In a preferred process for preparing such compositions, a liquid matrix is formed containing at least a major proportion, and preferably substantially all, of the liquid components, e.g., nonionic surfactant, the non-surface active liquid carriers and other optional liquid components, with the liquid components being thoroughly admixed by imparting shear agitation to this liquid combination. For example, rapid stirring with a mechanical stirrer may usefully be employed. While shear agitation is maintained, substantially all of any anionic surfactants and the solid form ingredients can be added. Agitation of the mixture is continued, and if necessary, can be increased at this point to form a solution or a uniform dispersion of insoluble solid phase particulates within the liquid phase. After some or all of the solid-form materials have been added to this agitated mixture, particles of any enzyme material to be included, e.g., enzyme prills, are incorporated. As a variation of the composition preparation procedure hereinbefore described, one or more of the solid components may be added to the agitated mixture as a solution or slurry of particles premixed with a minor portion of one or more of the liquid components. After addition of all of the composition components, agitation of the mixture is continued for a period of time sufficient to form compositions having the requisite viscosity and phase stability characteristics. Frequently this will involve agitation for a period of from about 30 to 60 minutes.

In an alternate embodiment for forming the liquid detergent compositions, the phenolic composition is first combined with one or more liquid components to form a phenolic composition premix, and this premix is added to a composition formulation containing a substantial portion, for example more than 50% by weight, more specifically, more than 70% by weight, and yet more specifically, more than 90% by weight, of the balance of components of the laundry detergent composition. For example, in the methodology described above, both the phenolic composition premix and the enzyme component are added at a final stage of component additions. In a further embodiment, the phenolic composition is encapsulated prior to addition to the detergent composition, the encapsulated phenolic composition is suspended in a structured liquid, and the suspension is added to a composition formulation containing a substantial portion of the balance of components of the laundry detergent composition.

The compositions of this invention, prepared as hereinbefore described, can be used to form aqueous washing solutions for use in the laundering of textile substrates such as fabrics. Generally, an effective amount of such compositions is added to water, preferably in a conventional fabric laundering automatic washing machine, to form such aqueous laundering solutions. The aqueous washing solution so formed is then contacted, preferably under agitation, with the fabrics to be laundered therewith. An effective amount of the liquid detergent compositions described herein is added to water to form aqueous laundering solutions comprising from about 500 to 7,000 ppm of the detergent composition in the aqueous washing solution. More preferably, from about 1,000 to 3,000 ppm of the detergent compositions described herein will be provided in aqueous washing solution.

Fabric Treatment Compositions / Rinse Added Fabric Softening Compositions

According to the invention, the phenolic composition is included in a fabric treatment composition. The fabric treatment composition may be comprised of at least one phenolic composition and a rinse added fabric softening composition ("RAFS;" also known as rinse added fabric conditioning compositions). Examples of typical rinse added softening compositions can be found in U.S. Provisional Patent Application Serial No. 60/687582 filed on October 8, 2004. The rinse added fabric softening compositions of the present invention may comprise (a) fabric softening active ("FSA") and (b) phenolic composition. The rinse added fabric softening composition may comprise from about 1% to about 90% by weight of the FSA, more preferably from about 5% to about 50% by weight of the FSA. The phenolic composition may be present in the rinse added fabric softening composition in an amount from about 0.5 ppb to about 10000 ppm, more preferably from about 0.5 ppm to about 1000 ppm.

In one embodiment of the invention, the fabric softening active is a quaternary ammonium compound suitable for softening fabric in a rinse step. In one embodiment, the FSA is formed from a reaction product of a fatty acid and an aminoalcohol obtaining mixtures of mono-, di-, and, in one embodiment, triester compounds. In another embodiment, the FSA comprises one or more softener quaternary ammonium compounds such, but not limited to, as a monoalkyquaternary ammonium compound, a diamido quaternary compound and a diester quaternary ammonium compound, or a combination thereof.

In one aspect of the invention, the FSA comprises a diester quaternary ammonium (hereinafter "DQA") compound composition. In certain embodiments of the present invention, the DQA compounds compositions also encompasses a description of diamido FSAs and FSAs with mixed amido and ester linkages as well as the aforementioned diester linkages, all herein referred to as DQA.

A first type of DQA ("DQA (1)") suitable as a FSA includes a compound comprising the formula:

$$\{R^{15}_{4-m} - N^{+} - [(CH_2)_n - Y - R^{16}]_m\}\ X^{-}$$

wherein each $R^{15}$ substituent is either hydrogen, a short chain $C_1$-$C_6$, preferably $C_1$-$C_3$ alkyl or hydroxyalkyl group, e.g.,

methyl (most preferred), ethyl, propyl, hydroxyethyl, and the like, poly ($C_{2-3}$ alkoxy), preferably polyethoxy, group, benzyl, or mixtures thereof, each m is 2 or 3; each n is from 1 to about 4, preferably 2; each Y is -O-(O)C-, -C(O)-O-, -NR-C(O)-, or -C(O)-NR- and it is acceptable for each Y to be the same or different; the sum of carbons in each $R^1$, plus one when Y is -O-(O)C- or -NR-C(O) -, is $C_{12}$-$C_{22}$, preferably $C_{14}$-$C_{20}$, with each $R^{16}$ being a hydrocarbyl, or substituted hydrocarbyl group; it is acceptable for $R^1$ to be unsaturated or saturated and branched or linear and preferably it is linear; it is acceptable for each $R^1$ to be the same or different and preferably these are the same; and $X^-$ can be any softener-compatible anion, preferably, chloride, bromide, methylsulfate, ethylsulfate, sulfate, phosphate, and nitrate, more preferably chloride or methyl sulfate. Preferred DQA compounds are typically made by reacting alkanolamines such as MDEA (methyldiethanolamine) and TEA (triethanolamine) with fatty acids. Some materials that typically result from such reactions include N,N-di(acyl-oxyethyl)-N,N-dimethylammonium chloride or N,N-di(acyl-oxyethyl)-N,N-methylhydroxyethylammonium methylsulfate wherein the acyl group is derived from animal fats, unsaturated, and polyunsaturated, fatty acids, e.g., tallow, hardened tallow, oleic acid, and/or partially hydrogenated fatty acids, derived from vegetable oils and/or partially hydrogenated vegetable oils, such as, canola oil, safflower oil, peanut oil, sunflower oil, corn oil, soybean oil, tall oil, rice bran oil, palm oil, etc.

Non-limiting examples of suitable fatty acids are listed in US Patent No. 5,759,990 at column 4, lines 45-66. In one embodiment, the FSA comprises other actives in addition to DQA (1) or DQA. In yet another embodiment, the FSA comprises only DQA (1) or DQA and is free or essentially free of any other quaternary ammonium compounds or other actives. In yet another embodiment, the FSA comprises the precursor amine that is used to produce the DQA.

In another aspect of the invention, the FSA comprises a compound, identified as DTTMAC comprising the formula:

$$[R^{17}_{4-m} - N^{(+)} - R^{18}_{m}]\ A-$$

wherein each m is 2 or 3, each $R^{18}$ is a $C_6$-$C_{22}$, preferably $C_{14}$-$C_{20}$, but no more than one being less than about $C_{12}$ and then the other is at least about 16, hydrocarbyl, or substituted hydrocarbyl substituent, preferably $C_{10}$-$C_{20}$ alkyl or alkenyl (unsaturated alkyl, including polyunsaturated alkyl, also referred to sometimes as "alkylene"), most preferably $C_{12}$-$C_{18}$ alkyl or alkenyl, and branch or unbranched. In one embodiment, the Iodine Value (IV) of the FSA is from about 1 to 70; each $R^{17}$ is H or a short chain $C_1$-$C_6$, preferably $C_1$-$C_3$ alkyl or hydroxyalkyl group, e.g., methyl (most preferred), ethyl, propyl, hydroxyethyl, and the like, benzyl, or $(R^{19}O)_{2-4}H$ where each $R^{19}$ is a $C_{1-6}$ alkylene group; and $A^-$ is a softener compatible anion, preferably, chloride, bromide, methylsulfate, ethylsulfate, sulfate, phosphate, or nitrate; more preferably chloride or methyl sulfate.

Examples of these FSAs include dialkydimethylammonium salts and dialkylenedimethylammonium salts such as ditallowdimethylammonium and ditallowdimethylammonium methylsulfate. Examples of commercially available dialkylenedimethylammonium salts usable in the present invention are di-hydrogenated tallow dimethyl ammonium chloride and ditallowdimethyl ammonium chloride available from Degussa under the trade names Adogen® 442 and Adogen® 470 respectively. In one embodiment, the FSA comprises other actives in addition to DTTMAC. In yet another embodiment, the FSA comprises only compounds of the DTTMAC and is free or essentially free of any other quaternary ammonium compounds or other actives.

In one embodiment, the FSA comprises an FSA described in U.S. Pat. Pub. No. 2004/0204337 A1, published Oct. 14, 2004 to Corona et al., from paragraphs 30 - 79. In another embodiment, the FSA is one described in U.S. Pat. Pub. No. 2004/0229769 A1, published Nov. 18, 2005, to Smith et al., on paragraphs 26 - 31; or U.S. Pat. No. 6,494,920, at column 1, line 51 *et seq.* detailing an "esterquat" or a quaternized fatty acid triethanolamine ester salt.

In one embodiment, the FSA is chosen from at least one of the following: ditallowoyloxyethyl dimethyl ammonium chloride, dihydrogenated-tallowoyloxyethyl dimethyl ammonium chloride, ditallow dimethyl ammonium chloride, ditallowoyloxyethyl dimethyl ammonium methyl sulfate, dihydrogenated-tallowoyloxyethyl dimethyl ammonium chloride, dihydrogenated-tallowoyloxyethyl dimethyl ammonium chloride, or combinations thereof.

In one embodiment, the FSA may also include amide containing compound compositions. Examples of diamide comprising compounds may include but not limited to methyl-bis(tallowamidoethyl)-2-hydroxyethylammonium methyl sulfate (available from Degussa under the trade names Varisoft 110 and Varisoft 222). An example of an amide-ester containing compound is N-[3-(stearoylamino)propyl]-N-[2-(stearoyloxy)ethoxy)ethyl)]-N-methylamine.

Another specific embodiment of the invention provides for a rinse added fabric softening composition further comprising a cationic starch. Cationic starches are disclosed in US 2004/0204337 A1. In one embodiment, the rinse added fabric softening composition comprises from about 0.1% to about 7% of cationic starch by weight of the fabric softening composition. In one embodiment, the cationic starch is HCP401 from National Starch.

<u>Exemplary Laundry Care Composition Formulations:</u>

Liquid Detergent Formulations:

Table A provides examples of liquid detergent formulations which include at least one phenolic composition of the present invention.

**Table A - Liquid Detergent Formulations Comprising Phenolic Composition**

| Ingredient | 1a | 1b | 1c | 1d | 1e | 1f [4] |
|---|---|---|---|---|---|---|
| | wt % | wt % | wt % | wt % | wt % | wt % |
| sodium alkyl ether sulfate | 14.4% | 14.4% | | 9.2% | 5.4% | |
| linear alkylbenzene sulfonic acid | 4.4% | 4.4% | 12.2% | 5.7% | 1.3% | 22.0% |
| alkyl ethoxylate | 2.2% | 2.2% | 8.8% | 8.1% | 3.4% | 18.0% |
| amine oxide | 0.7% | 0.7% | 1.5% | | | |
| citric acid | 2.0% | 2.0% | 3.4% | 1.9% | 1.0% | 1.6% |
| fatty acid | 3.0% | 3.0% | 8.3% | | | 16.0% |
| Protease | 1.0% | 1.0% | 0.7% | 1.0% | | 2.5% |
| Amylase | 0.2% | 0.2% | 0.2% | | | 0.3% |
| Lipase | | | | 0.2% | | |
| Borax | 1.5% | 1.5% | 2.4% | 2.9% | | |
| calcium and sodium formate | 0.2% | 0.2% | | | | |
| formic acid | | | | | | 1.1% |
| amine ethoxylate polymers | 1.8% | 1.8% | 2.1% | | | 3.2% |
| sodium polyacrylate | | | | | 0.2% | |
| sodium polyacrylate copolymer | | | | 0.6% | | |
| DTPA[1] | 0.1% | 0.1% | | | | 0.9% |
| DTPMP[2] | | | 0.3% | | | |
| EDTA[3] | | | | | 0.1% | |
| fluorescent whitening agent | 0.15% | 0.15% | 0.2% | 0.12% | 0.12% | 0.2% |
| Ethanol | 2.5% | 2.5% | 1.4% | 1.5% | | |
| propanediol | 6.6% | 6.6% | 4.9% | 4.0% | | 15.7% |
| Sorbitol | | | | 4.0% | | |
| ethanolamine | 1.5% | 1.5% | 0.8% | 0.1% | | 11.0% |
| sodium hydroxide | 3.0% | 3.0% | 4.9% | 1.9% | 1.0% | |
| sodium cumene sulfonate | | | 2.0% | | | |
| silicone suds suppressor | | | 0.01% | | | |
| Perfume | 0.3% | 0.3% | 0.7% | 0.3% | 0.4% | 0.6% |
| Phenolic Composition | 0.013% | 0.001% | 0.1% | 1% | 0.0005% | 0.001% |
| Water | balance 100.0% | Balance 100.0% | balance 100.0% | balance 100.0% | balance 100.0% | Balance 100.0% |

[1] diethylenetriaminepentaacetic acid, sodium salt
[2] diethylenetriaminepentakismethylenephosphonic acid, sodium salt
[3] ethylenediaminetetraacetic acid, sodium salt
[4] a compact formula, packaged as a unitized dose in polyvinyl alcohol film

Granular Detergent Formulations:

Table B provides examples of granular detergent formulations which include at least one phenolic composition of the present invention.

**Table B - Granular Detergent Formulations Comprising Phenolic Composition**

| Ingredient | 2a | 2b | 2c | 2d | 2e |
|---|---|---|---|---|---|
| | wt % | wt % | wt % | wt % | wt % |
| Na linear alkylbenzene sulfonate | 3.4% | 3.3% | 11.0% | 3.4% | 3.3% |
| Na alkylsulfate | 4.0% | 4.1% | | 4.0% | 4.1% |
| Na alkyl sulfate (branched) | 9.4% | 9.6% | | 9.4% | 9.6% |
| alkyl ethoxylate | | | 3.5% | | |
| type A zeolite | 37.4% | 35.4% | 26.8% | 37.4% | 35.4% |
| sodium carbonate | 22.3% | 22.5% | 35.9% | 22.3% | 22.5% |
| sodium sulfate | 1.0% | | 18.8% | 1.0% | |
| sodium silicate | | | 2.2% | | |
| Protease | 0.1% | 0.2% | | 0.1% | 0.2% |
| sodium polyacrylate | 1.0% | 1.2% | 0.7% | 1.0% | 1.2% |
| carboxymethylcellulose | | | 0.1% | | |
| PEG 600 | | 0.5% | | | 0.5% |
| PEG 4000 | | 2.2% | | | 2.2% |
| DTPA | 0.7% | 0.6% | | 0.7% | 0.6% |
| fluorescent whitening agent | 0.1% | 0.1% | 0.1% | 0.1% | 0.1% |
| sodium percarbonate | | 5.0% | | | 5.0% |
| sodium nonanoyloxybenzenesulfonate | | 5.3% | | | 5.3% |
| silicone suds suppressor | 0.02% | 0.02% | | 0.02% | 0.02% |
| Perfume | 0.3% | 0.3% | 0.2% | 0.3% | 0.3% |
| Phenolic composition | 0.004% | 0.1% | 0.04% | 1% | 0.02% |
| water and miscellaneous | balance 100.0% | balance 100.0% | balance 100.0% | balance 100.0% | Balance 100.0% |

Fabric Treatment Compositions:

Table C provides examples of liquid fabric treatment compositions which include at least one phenolic composition of the present invention.

**Table C - Liquid Fabric Treatment Compositions Comprising Phenolic Composition**

| Ingredients | a | b | c | d |
|---|---|---|---|---|
| Fabric Softening Active [a] | 13.70% | 13.70% | 13.70% | 13.70% |
| Ethanol | 2.14% | 2.14% | 2.14% | 2.14% |
| Cationic Starch [b] | 2.17% | 2.17% | 2.17% | 2.17% |
| Perfume | 1.45% | 1.45% | 1.45% | 1.45% |
| Phase Stabilizing Polymer [c] | 0.21% | 0.21% | 0.21% | 0.21% |
| Calcium Chloride | 0.147% | 0.147% | 0.147% | 0.147% |
| DTPA [d] | 0.007% | 0.007% | 0.007% | 0.007% |
| Preservative [e] | 5 ppm | 5 ppm | 5 ppm | 5 ppm |
| Antifoam [f] | 0.015% | 0.015% | 0.015% | 0.015% |
| Phenolic Composition | 0.1% | 0.01% | 1% | 0.0001% |
| Tinopal CBS-X [g] | 0.2 | 0.2 | 0.2 | 0.2 |
| Ethoquad C/25 [h] | 0.26 | 0.26 | 0.26 | 0.26 |
| Ammonium Chloride | 0.1% | 0.1% | 0.1% | 0.1% |

(continued)

| Ingredients | a | b | c | d |
|---|---|---|---|---|
| Hydrochloric Acid | 0.012 % | 0.012 % | 0.012 % | 0.012 % |
| Deionized Water | Balance | Balance | Balance | Balance |

[a] N,N-di(tallowoyloxyethyl)-N,N-dimethylammonium chloride.
[b] Cationic starch based on common maize starch or potato starch, containing 25% to 95% amylose and a degree of substitution of from 0.02 to 0.09, and having a viscosity measured as Water Fluidity having a value from 50 to 84.
[c] Copolymer of ethylene oxide and terephthalate having the formula described in US 5,574,179 at col.15, lines 1-5, wherein each X is methyl, each n is 40, u is 4, each $R^1$ is essentially 1,4-phenylene moieties, each $R^2$ is essentially ethylene, 1,2-propylene moieties, or mixtures thereof.
[d] Diethylenetriaminepentaacetic acid.
[e] KATHON® CG available from Rohm and Haas Co.
[f] Silicone antifoam agent available from Dow Corning Corp. under the trade name DC2310.
[g] Disodium 4,4'-bis-(2-sulfostyryl) biphenyl, available from Ciba Specialty Chemicals.
[h] Cocomethyl ethoxylated [15] ammonium chloride, available from Akzo Nobel.

Textile substrates treated with the laundry care composition(s) comprised of the phenolic composition of the present invention may be comprised of synthetic fibers, natural fibers, or combinations of synthetic and natural fibers. Synthetic fibers include, for example, polyester, acrylic, polyamide, polyolefin, polyaramid, polyurethane, regenerated cellulose (i.e., rayon), and blends thereof. The term "polyamide" is intended to describe any long-chain polymer having recurring amide groups (--NH-CO--) as an integral part of the polymer chain. Examples of polyamides include nylon 6; nylon 6, 6; nylon 1, 1; and nylon 6, 10. The term "polyester" is intended to describe any long-chain polymer having recurring ester groups (--C(O)-O--). Examples of polyesters include aromatic polyesters, such as polyethylene terephthalate (PET), polybutylene terephthalate (PBT), polytrimethylene terephthalate (PTT), and polytriphenylene terephthalate, and aliphatic polyesters, such as polylactic acid (PLA). "Polyolefin" includes, for example, polypropylene, polyethylene, and combinations thereof. "Polyaramid" includes, for example, poly-p-phenyleneteraphthalamid (i.e., Kevlar®), poly-m-phenyleneteraphthalamid (i.e., Nomex®), and combinations thereof. Natural fibers include, for example, wool, cotton, flax, and blends thereof.

The textile substrate may be of any variety, including but not limited to, woven fabric, knitted fabric, nonwoven fabric, or combinations thereof. The textile substrate may optionally be colored by a variety of dyeing techniques, such as high temperature jet dyeing with disperse dyes, vat dyeing, thermosol dyeing, pad dyeing, transfer printing, screen printing, or any other technique that is common in the art for comparable textile products. The yarns or fibers comprising the textile substrate may optionally be dyed by suitable methods prior to fabric formation, such as, for instance, by package dyeing or solution dyeing.

Textile substrates include, for example, articles of apparel, such as outerwear (e.g., rainwear), workwear (e.g., uniforms), fashion apparel (e.g., shirts, pants, and other garments); drapery; napery (e.g., table linens and napkins); residential upholstery; commercial upholstery; automotive upholstery; wall coverings; floorcovering articles (e.g., carpets, rugs and mats); human bedding (e.g., mattresses, mattress covers, and the like); pet bedding; outdoor fabric (e.g., outdoor furniture, awnings, boat covers, and grill covers); medical dressings (e.g., fabrics for use in wound care); and any other article capable of possessing discoloration and wherein it is desirable to control (e.g. prevent, remove, and/or improve) said discoloration.

## EXAMPLES

The following examples are for the purpose of illustration of the invention only and are not intended to limit the scope of the present invention in any manner whatsoever.

### Example 1: Synthesis of Phenolic Compositions

*Compound 1: Methyl 3-[3-(*tert*-butyl)-4-hydroxy-5-tolyl]propionate*

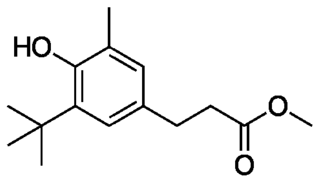

A 1000mL 4-neck round bottom flask was affixed with temperature probe, overhead stirring and a reflux condenser under a nitrogen atmosphere. To the reaction flask was charged, 100g 2-[2-(2-{3-[3-(*tert*-Butyl)-4-hydroxy-5-tolyl]propionoxy}ethoxy)ethoxy]ethyl 3-[3-(*tert*-butyl)-4-hydroxy-5-tolyl]propionate (Irganox 245 available from BASF), 6g of a 30% sodium methoxide solution in methanol and 200g of methanol. The reaction was stirred at 20-25°C for 16 hours and then neutralized with 2g of acetic acid. To the reaction flask was added 100mL of distilled water and 100mL of hexane, then the contents of the flask were transferred to a separatory funnel followed by an additional 300mL of distilled water. The phases were separated and the organic layer was collected and dried under reduced pressure to obtain methyl 3-[3-(*tert*-butyl)-4-hydroxy-5-tolyl]propionate as an oil that solidified upon standing.

*Composition 1*

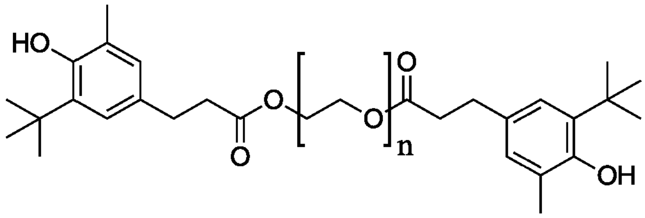

wherein average n≈4.1

A 100mL 3-neck round bottom flask was affixed with a temperature probe, overhead stirring and a dean-stark trap with a condenser under a nitrogen atmosphere. To the reaction flask was charged, 20g of methyl 3-[3-(tert-butyl)-4-hydroxy-5-tolyl]propionate, 8g of PEG 200 and 0.4g dibutyltin oxide. The reaction was heated to 240°C and stirred until it was judged complete by TLC analysis (about 18-24 hours). The product was obtained as a brown oil. The product was a composition containing a distribution of phenolic compounds encompassed by the formula above, and the value of n is equal to the average number of ethylene oxide repeat units of the phenolic compounds present in the composition.

*Composition 2*

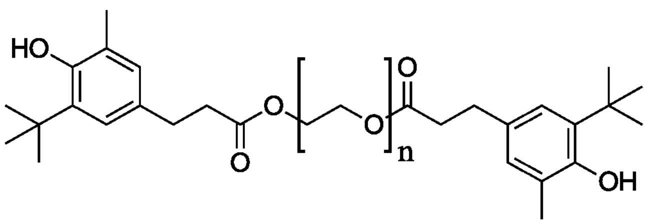

wherein average n≈6.5

The same procedure as in Composition 1, except 12g of PEG 300 was used in place of the PEG 200. The product was obtained as a dark yellow oil. The product was a composition containing a distribution of phenolic compounds encompassed by the formula above, and the value of n is equal to the average number of ethylene oxide repeat units of the phenolic compounds present in the composition.

*Composition 3*

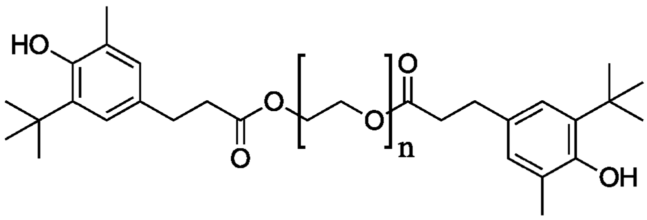

wherein average n≈13.2

The same procedure as in Composition 1, except 24g of PEG 600 was used in place of the PEG 200. The product was obtained as a dark yellow oil. The product was a composition containing a distribution of phenolic compounds encompassed by the formula above, and the value of n is equal to the average number of ethylene oxide repeat units of the phenolic compounds present in the composition.

*Composition 4*

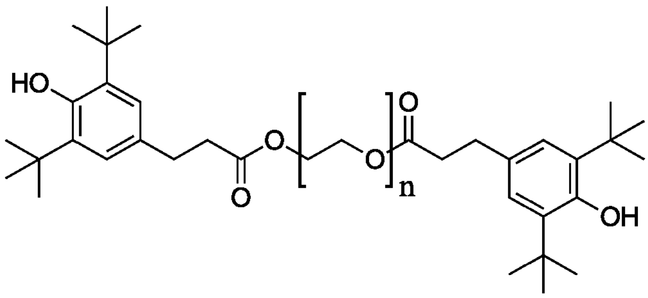

wherein average n≈4.1

A 3-neck 100mL round bottom flask was affixed with temperature probe, overhead stirring and a dean stark trap with a condenser under nitrogen atmosphere. To the reaction flask was charged, 23.4g of methyl 3-[3,5-bis(*tert*-butyl)-4-hydroxyphenyl]propionate, 8g of PEG 200 and 0.4g dibutyltin oxide. The reaction was heated to 140°C until complete as monitored by TLC. The product was a composition containing a distribution of phenolic compounds encompassed by the formula above, and the value of n is equal to the average number of ethylene oxide repeat units of the phenolic compounds present in the composition.

*Composition 5*

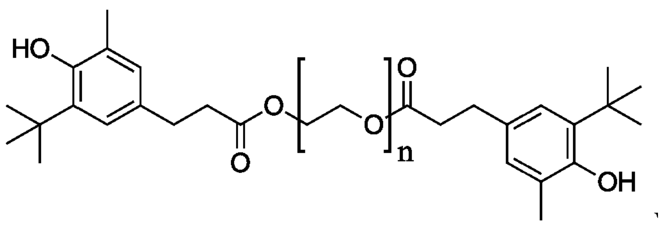

wherein average n≈9

A 100mL 3-neck round bottom flask was affixed with a temperature probe, overhead stirring and a dean-stark trap with a condenser under a nitrogen atmosphere. To the reaction flask was charged, 20g of methyl 3-[3-(*tert*-butyl)-4-hydroxy-5-tolyl]propionate, 16g of PEG 400 and 0.4g sodium acetate. The reaction was heated to 100°C and vacuum pulled for 1 hour to remove residual water. The reaction mixture was then heated to 160°C and stirred until it was judged complete by TLC analysis. The product was cooled and transferred to a jar. The product was a composition containing a distribution of phenolic compounds encompassed by the formula above, and the value of n is equal to the average number of ethylene oxide repeat units of the phenolic compounds present in the composition.

*Compound 2*

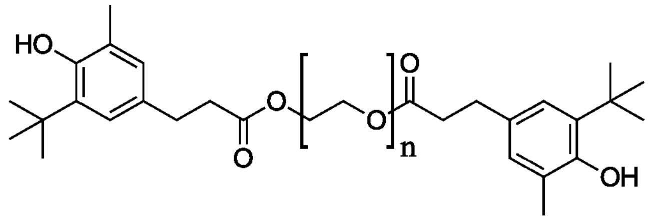

wherein n=4

A 100mL 3-neck round bottom flask was affixed with a temperature probe, overhead stirring and a dean-stark trap with a condenser under a nitrogen atmosphere. To the reaction flask was charged, 20g of methyl 3-[3-(*tert*-butyl)-4-hydroxy-5-tolyl]propionate, 7.2g of tetraethylene glycol (available from Sigma-Aldrich) and 0.4g sodium acetate. The reaction was heated to 100°C and vacuum pulled for 1 hour to remove residual water. The reaction mixture was then heated to 160°C and stirred until it was judged complete by TLC analysis. The product was cooled and transferred to a jar.

**Example 2: Assessment of Malodor Reduction**

In order to test the efficiency of the antioxidants for malodor reduction, an artificial body soil (ABS) was prepared. The artificial body soil is a modified version of the soil from ASTM D4265-21. To prepare the ABS, the components of Table 1 were combined in an 8 oz. jar and heated to 70°C until fully solubilized.

Table 1. Artificial Body Soil Composition

| Material | Weight Percent Composition (%) |
|---|---|
| White coconut oil, refined (LouAna) | 7.5 |
| Stearic acid (TCI) | 2.5 |
| Squalene oil (Spectrum Chemical) | 2.5 |
| Cholesterol powder (TCI) | 2.5 |
| Olive oil (Bertolli) | 10 |
| Palmitic acid (TCI) | 5 |
| Paraffin oil (Spectrum Chemical) | 5 |
| Vegetable oil (GreatValue) | 10 |
| Oleic Acid (Emery Oleochemicals) | 5 |
| Dipropylene Glycol Monomethyl Ether (TCI) | 50 |

Fabric preparation:

50/50 Cotton/poly interlock fabric (Style 7439OB, Testfabrics, Inc.) was cut into 2in x 5in swatches. Cut swatches were dosed with three equally spaced 0.36mL portions of artificial body soil composition. Fabrics were then sealed together in a mylar bag and incubated at 37°C for four to seven days prior to wash treatment.

Wash Water:

The wash water was used at 7 gpg hardness and 800 ng/mL of copper by dissolving the components listed in Table 2 in DI water.

Table 2. Wash Water Composition

| Component | Amount/L of Wash Water |
|---|---|
| Calcium Chloride | 0.0933g |
| Magnesium Chloride | 0.0267g |

(continued)

| Component | Amount/L of Wash Water |
|---|---|
| Copper (II) Chloride | 0.0017g |

Antioxidant Solution Preparation:

Antioxidant solution was prepared by dissolving the antioxidant compound to be tested in methanol at 0.5% w/w. The antioxidant solution was dosed into the wash water (500 mL) using a micropipettor according to Table 3 to obtain the desired concentration of antioxidant in wash water required for the testing.

Table 3. Antioxidant Solution Addition to Wash Water and Corresponding Concentration of Antioxidant in Wash Water

| Amount of antioxidant solution added to wash water | Concentration of antioxidant in wash water |
|---|---|
| 1.0 mL | 8 ppm |
| 0.5 mL | 4 ppm |
| 0.25 mL | 2 ppm |

**Wash and rinse method:**

To 1L wash beaker was added 0.4g Tide Free and Gentle detergent, 500mL of wash water (as prepared in Table 2) and the antioxidant solution as described in Table 3. The wash beaker was placed in a tergetometer. To the wash beaker was added two incubated fabric swatches (4g) followed by one 11in x 11in 50/50 Cotton/Poly interlock knit clean fabric swatch (16g, Style 7439OB, Testfabrics, Inc.), for a total liquor to fabric ratio of 25:1. The fabrics were then washed in the tergotometer for 15 minutes and spun dried for one minute in a spin dryer (Mini Spin Dryer Model LASD-1 available from The Laundry Alternative, Inc.). Fabrics were then transferred from the spin dryer to 500mL of fresh wash water (as prepared above) and rinsed for 15 minutes in the tergetometer. Fabrics were then spun dry for 1 minute in the spin dryer and transferred to a dryer to dry on medium heat for 1 hour. Once dried, the artificial body soil treated and washed fabrics were cut in half (1in x 5in swatches), rolled, and put in gas chromatography vials for headspace analysis. The tests were run in triplicate and an average malodor reduction percent was calculated.

Malodor Analysis on Fabric Using GCMS Analysis

The GC data tracks the malodor marker concentration in the headspace that occurs from the oxidative breakdown of the ABS. The marker that is tracked for this breakdown of the ABS is 3-methyl-2-butenal.

The percent malodor reduction is determined by GCMS using an Agilent Intuvo 9000 GC equipped with an Agilent 7000D GC/TQ mass spectrometer and a Trajan PAL 3 RTC sampler equipped with a solid phase micro-extraction (SPME) probe. A calibration standard of the tracked malodor marker, 3-methyl-2-Butenal (107-86-8) is prepared by dissolving a known weight of mineral oil (CAS 8020-83-5). Fabrics are cut into uniform 1 inch by 5 inch pieces and placed in 10 mL headspace crimp vials. Vials are equilibrated greater than 12 hours before analysis. The following settings are used in the auto sampler: 90°C incubation temperature, 90 min incubation time, VT25 sample tray type, 22 mm vial penetration, 10 min extraction time, 50 mm injection penetration and 3 min desorption time. The column is an Agilent DB-624 Ultra Inert, 60 m x 250 μm x 1.4 μm (Part number 122-1364UI-INT) . The following settings are used for the Front Split/Splitless inlet helium: split mode, 250°C temperature, 39.3 psi pressure, 30.5 mL/min total flow, 3 mL/min septum purge flow, 10:1 split ratio and18.5 min GC run time. The follow settings are used in the oven: 35°C initial temperature, hold 1 min at 35°C then 30°C/min heating program, 260°C final temperature and 10 min hold time at 260°C. The extracted ion chromatogram for 3-methyl-2-butenal includes Quantifier: 84, Qualifier: 55, Ratio: 35.7.

Results:

Following the test method as laid out above, the antioxidants were tested for the reduction of the malodor marker. All tests were run in triplicate and the average of the three swatches was taken. The difference in the counts between the control vs the antioxidant containing composition is used to calculate the percent malodor marker reduction. The percent malodor marker reduction was calculated by the following equation:

$$\% \text{ malodor marker reduction} = 100 - (\text{Marker Amount}_{Test}/\text{Marker Amount}_{Control} * 100)$$

**Comparison of Substitution Pattern:**

A comparison of the inventive compounds to antioxidants with di-tert-butyl substitutions on the phenolic rings of the antioxidants was made. Each antioxidant was run through the wash test at varying concentrations through the wash (2,4,8 ppm concentration of AO in wash water) and results are listed in Table 4. The preferred phenolic compositions of the invention (Compositions 1-3) are compared to the control (detergent nil antioxidant) and a comparative example (Composition 4) that does not contain the preferred substitution pattern on the hindered phenol moiety.

Table 4. Wash test results

| Sample | Concentartion in wash water | Molarity in wash water (mols/L) | Average Malodor Marker Amount | % Malodor Marker Reduction |
|---|---|---|---|---|
| Control (detergent only nil antioxidant) | 0 ppm | n/a | 13245581 | n/a |
| Composition 1 | 8 ppm | 1.2x10-5 | 1228722 | 90.72 |
| Composition 1 | 4 ppm | 6.1x10-6 | 1663349 | 87.44 |
| Composition 1 | 2 ppm | 3.1x10-6 | 6714461 | 49.31 |
| Composition 2 | 8 ppm | 1.1x10-5 | 1621135 | 87.76 |
| Composition 2 | 4 ppm | 5.3x10-6 | 8066570 | 39.10 |
| Composition 2 | 2 ppm | 2.7x10-6 | 11581964 | 12.56 |
| Composition 3 | 8 ppm | 7.6x10-6 | 3760097 | 71.61 |
| Composition 3 | 4 ppm | 3.8x10-6 | 5551715 | 58.09 |
| Composition 3 | 2 ppm | 1.9x10-6 | 8818964 | 33.42 |
| Composition 4 | 8 ppm | 1.1x10-5 | 2465462 | 81.39 |
| Composition 4 | 4 ppm | 5.4x10-6 | 6405847 | 51.64 |
| Composition 4 | 2 ppm | 2.7x10-6 | 12259647 | 7.44 |

As one can see from the table above, all inventive phenolic compositions perform better than the control at reducing malodor and the preferred compositions, especially composition 1, which is the most preferred composition, perform as good or better than the comparative composition 4 at reducing the level of malodor marker.

Comparison of plurality of molecules vs single compound:

A comparison for malodor reduction of the inventive compositions to Irganox 245, which is a commercially available antioxidant from BASF that contains a 3EO chain linker as a single molecule, was made. Each antioxidant was run in a wash test at varying concentrations through the wash (2,4,8 ppm concentration of AO in wash water) and results are listed in Table 5. The preferred phenolic composition of the invention is compared to the control (detergent nil antioxidant) and a comparative example (Irganox 245) that does not contain a plurality of molecules was made.

Table 5. Wash test results

| Sample | Concentration in wash water | % Malodor marker reduction |
|---|---|---|
| Control (detergent only nil antioxidant) | n/a | n/a |
| Composition 1 | 8 ppm | 89.4 |
| Composition 1 | 4 ppm | 80.7 |
| Composition 1 | 2 ppm | 51.0 |
| Irganox 245 | 8 ppm | 89.5 |

(continued)

| Sample | Concentration in wash water | % Malodor marker reduction |
|---|---|---|
| Control (detergent only nil antioxidant) | n/a | n/a |
| Irganox 245 | 4 ppm | 74.4 |
| Irganox 245 | 2 ppm | 44.0 |

As one can see from the table above, the inventive phenolic composition 1 performs better than the control at reducing malodor and the preferred composition 1 performs better than Irganox 245, which is a single molecule, especially at lower concentrations in the wash water.

**Assessment of stability**

A comparison of the stability of the antioxidants in standard AATCC detergent was made to highlight the benefit of having a plurality of molecules. Due to the highly crystalline nature of many commercially available antioxidants, they tend to be incompatible in detergent and will crystallize out of the detergent over time. The inventive compounds of this invention show improvements in remaining stable in liquid detergent.

The antioxidants in table 6 were combined with AATCC HE liquid detergent without optical brighteners (available from Test Fabrics, Inc., Item #0501001) in a 20 mL glass scintillation vial. The vials were placed in a sonicator for 1 hour, then heated to 80°C for 30 minutes, whereby all samples were completely dissolved. The samples were removed from the oven, swirled briefly and hand then allowed to cool to room temperature. When the vials were cooled down to room temperature, the bottom of the vials were scratched with a metal spatula a few times to aide in the crystallization process, which can take a long time if left undisturbed. After an additional 3 days, observations were recorded as crystallization present or not present in Table 6.

Table 6.

| Compound | Loading in AATCC detergent (ppm) | Precipitation Seen |
|---|---|---|
| Irganox 245 | 500 | No |
| | 1000 | No |
| | 5000 | Yes |
| | 10000 | Yes |
| Composition 1 | 500 | No |
| | 1000 | No |
| | 5000 | No |
| | 10000 | No |
| Composition 5 | 500 | No |
| | 1000 | No |
| | 5000 | No |
| | 10000 | No |
| Composition 3 | 500 | No |
| | 1000 | No |
| | 5000 | No |
| | 10000 | No |
| 50/50 blend of Irganox 245 and Compound 6 | 500 | No |
| | 1000 | No |
| | 5000 | No |
| | 10000 | No |

As one can see from the results in table 6, a plurality of molecules is beneficial to prevent precipitation of the antioxidant from the detergent formulation over time. This allows the formulator to incorporate higher levels of antioxidant into the detergent if desired and not be concerned about incompatibilities.

**Lower concentration of antioxidant in detergent test:**

In order to show the malodor reduction efficiency of the inventive compositions at lower concentrations of phenolic antioxidant, a slight variation to the procedure was undertaken. The wash method was the same except adjustments to the copper loading in the wash water and the antioxidant use levels were made. For the experiment, the antioxidant level was reduced to 700ppm in Tide Free and Gentle detergent (which equates to 0.56 ppm antioxidant concentration in wash water) and copper in wash water was used at a level of 100 ng Cu/g of wash water. Table 7 shows the results of the test.

Table 7. Wash test results with reduced copper and antioxidant levels

| Sample | Concentration AO in wash water | % Malodor Marker Reduction |
|---|---|---|
| Control (detergent only nil antioxidant) | 0 ppm | n/a |
| Composition 1 | 0.56 ppm | 69.8 |
| Composition 2 | 0.56 ppm | 82.4 |
| Composition 3 | 0.56 ppm | 71.3 |

As one can see from Table 7, All inventive compositions perform well in reducing malodor markers on fabric with reduced loading levels through the wash.

The compositions and methods of the appended claims are not limited in scope by the specific compositions and methods described herein, which are intended as illustrations of a few aspects of the claims. The term "comprising" and variations thereof as used herein is used synonymously with the term "including" and variations thereof and are open, non-limiting terms. Although the terms "comprising" and "including" have been used herein to describe various embodiments, the terms "consisting essentially of" and "consisting of" can be used in place of "comprising" and "including" to provide for more specific embodiments of the invention and are also disclosed. Other than in the examples, or where otherwise noted, all numbers expressing quantities of ingredients, reaction conditions, and so forth used in the specification and claims are to be understood at the very least, to be construed in light of the number of significant digits and ordinary rounding approaches.

## Claims

**1.** A laundry care composition comprising:

a) a surfactant; and
b) a phenolic composition comprising:

i) a first phenolic compound having the formula:

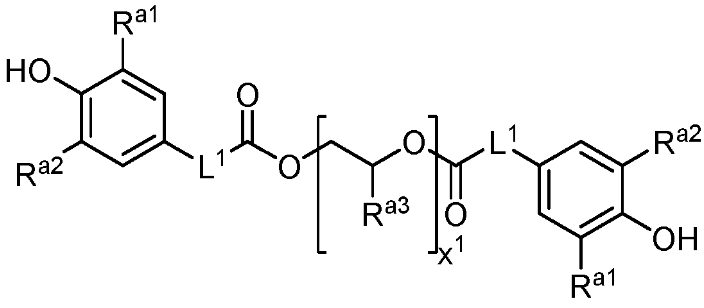

,

wherein $L^1$ is selected from null, $-CH_2-$, $-CH_2CH_2-$, and $-CH_2CH_2CH_2-$,
$x^1$ is an integer from 2-50;

$R^{a1}$ is a $C_{1-8}$ alkyl group;
$R^{a2}$ is a $C_{1-8}$ alkyl group;
$R^{a3}$ is H, $CH_3$, or $CH_2CH_3$;

ii) a second phenolic compound having the formula:

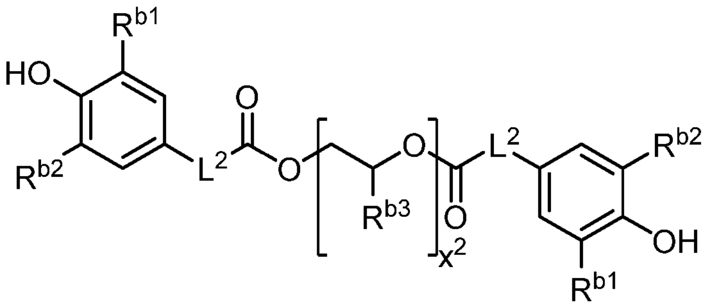
,

wherein $L^2$ is selected from null, $-CH_2-$, $-CH_2CH_2-$, and $-CH_2CH_2CH_2-$,
$x^2$ is an integer from 2-50; provided that $x^1$ and $x^2$ are not the same;
$R^{b1}$ is a $C_{1-8}$ alkyl group;
$R^{b2}$ is a $C_{1-8}$ alkyl group; and
$R^{b3}$ is H, $CH_3$, or $CH_2CH_3$.

**2.** The laundry care composition according to claim 1, wherein the phenolic composition is present in an amount from 0.0001 wt.% - 1.0 wt.% relative to the total weight of the laundry care composition.

**3.** The laundry care composition according to claim 1, wherein $R^{a1}$ and $R^{a2}$ are not the same.

**4.** The laundry care composition according to claim 1, wherein $R^{b1}$ and $R^{a2}$ are not the same.

**5.** The laundry care composition according to claim 1, wherein $R^{a2}$ and $R^{b2}$ are each $C_{3-5}$ alkyl, preferably wherein $R^{a2}$ and $R^{b2}$ are t-butyl.

**6.** The laundry care composition according to claim 1, wherein $R^{a1}$ and $R^{b1}$ are each a linear $C_{1-8}$ alkyl group, preferably wherein $R^{a1}$ and $R^{b1}$ are each a linear $C_{1-4}$ alkyl group, more preferably wherein $R^{a1}$ and $R^{b1}$ are each methyl, or $R^{a1}$ and $R^{b1}$ are each ethyl.

**7.** The laundry care composition according to claim 1, wherein $L^1$ is $-CH_2CH_2-$ or $-CH_2CH_2CH_2-$.

**8.** The laundry care composition according to claim 1, wherein $L^2$ is $-CH_2CH_2-$ or $-CH_2CH_2CH_2-$.

**9.** The laundry care composition according to claim 1, wherein $x^1$ is an integer from 4-25.

**10.** The laundry care composition according to claim 1, wherein $x^2$ is an integer from 2-25.

**11.** The laundry care composition according to claim 1, wherein $R^{a3}$ and $R^{b3}$ are each H.

**12.** The laundry care composition according to claim 1, wherein the molar ratio of the first phenolic compound to the second phenolic compound is from 50:1 to 1:50.

**13.** The laundry care composition according to claim 1, further comprising a third phenolic compound having the formula:

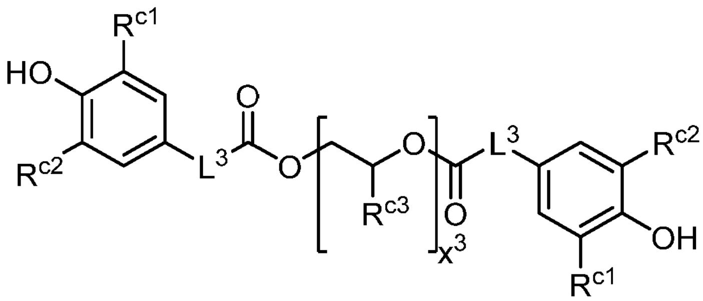

wherein $L^3$ is selected from null, $-CH_2-$, $-CH_2CH_2-$, and $-CH_2CH_2CH_2-$,
$x^3$ is an integer from 2-50; provided that none of $x^1$, $x^2$, and $x^3$ are the same;
$R^{c1}$ is a $C_{1-8}$ alkyl group;
$R^{c2}$ is a $C_{1-8}$ alkyl group; and
$R^{c3}$ is H, $CH_3$, or $CH_2CH_3$;

preferably further comprising a fourth phenolic compound having the formula:

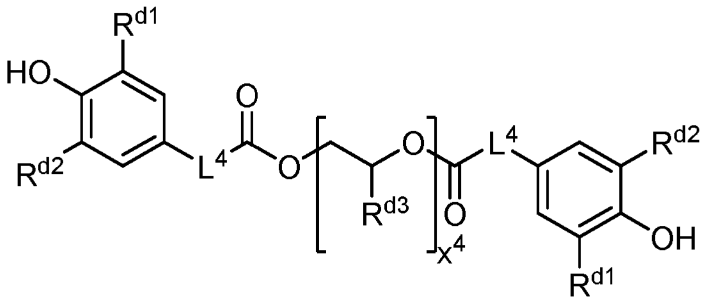

wherein $L^4$ is selected from null, $-CH_2-$, $-CH_2CH_2-$, and $-CH_2CH_2CH_2-$,
$x^4$ is an integer from 2-50; provided that none of $x^1$, $x^2$, $x^3$, and $x^4$ are the same;
$R^{d1}$ is a $C_{1-8}$ alkyl group;
$R^{d2}$ is a $C_{1-8}$ alkyl group; and
$R^{d3}$ is H, $CH_3$, or $CH_2CH_3$.

**14.** A method of laundering an article comprising contacting the article with the composition according to claim 1, preferably wherein the article is contacted with the composition in the presence of water.

**15.** A method of manufacturing a laundry care composition, comprising combining a water-soluble carrier, a surfactant and a phenolic composition, said phenolic composition comprising:

a first phenolic compound having the formula:

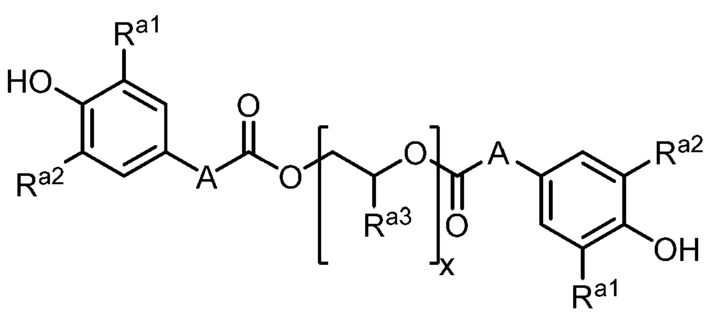
,

wherein A is selected from null, $-CH_2-$, $-CH_2CH_2-$, and $-CH_2CH_2CH_2-$,
x is an integer from 2-50;
$R^{a1}$ is a $C_{1-8}$ alkyl group;

$R^{a2}$ is a $C_{1-8}$ alkyl group;
$R^{a3}$ is H, $CH_3$, or $CH_2CH_3$; and

a second phenolic compound having the formula:

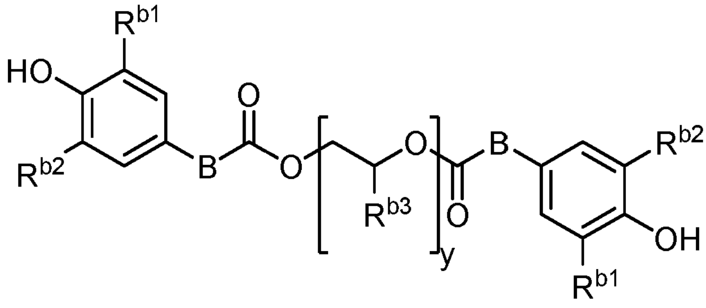

,

wherein B is selected from null, $-CH_2-$, $-CH_2CH_2-$, and $-CH_2CH_2CH_2-$,
y is an integer from 2-50; provided that x and y are not the same;
$R^{b1}$ is $C_{1-8}$ alkyl group;
$R^{b2}$ is a $C_{1-8}$ alkyl group; and
$R^{b3}$ is H, $CH_3$, or $CH_2CH_3$.

**Patentansprüche**

**1.** Wäschepflegezusammensetzung, umfassend:

a) ein Tensid; und
b) eine Phenolzusammensetzung, die Folgendes umfasst:

i) eine erste Phenolverbindung mit der folgenden Formel:

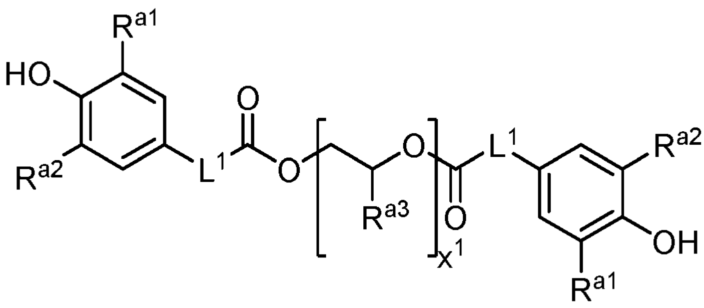

,

wobei $L^1$ ausgewählt ist aus Null, $-CH_2-$, $-CH_2CH_2-$ und $-CH_2CH_2CH_2-$,
$x^1$ eine ganze Zahl von 2-50 ist;
$R^{a1}$ eine $C_{1-8}$-Alkylgruppe ist;
$R^{a2}$ eine $C_{1-8}$-Alkylgruppe ist;
$R^{a3}$ H, $CH_3$ oder $CH_2CH_3$ ist;

ii) eine zweite Phenolverbindung mit der folgenden Formel:

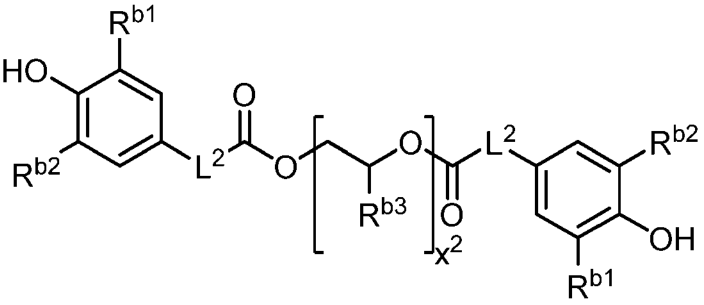

,

wobei $L^2$ ausgewählt ist aus Null, $-CH_2-$, $-CH_2CH_2-$ und $-CH_2CH_2CH_2-$,
$x^2$ eine ganze Zahl von 2-50 ist; vorausgesetzt, dass $x^1$ und $x^2$ nicht gleich sind;
$R^{b1}$ eine $C_{1-8}$-Alkylgruppe ist;
$R^{b2}$ eine $C_{1-8}$-Alkylgruppe ist; und
$R^{b3}$ H, $CH_3$ oder $CH_2CH_3$ ist.

2. Wäschepflegezusammensetzung nach Anspruch 1, wobei die Phenolzusammensetzung in einer Menge von 0,0001 Gew.-% - 1,0 Gew.-%, bezogen auf das Gesamtgewicht der Wäschepflegezusammensetzung, vorliegt.

3. Wäschepflegezusammensetzung nach Anspruch 1, wobei $R^{a1}$ und $R^{a2}$ nicht gleich sind.

4. Wäschepflegezusammensetzung nach Anspruch 1, wobei $R^{b1}$ und $R^{a2}$ nicht gleich sind.

5. Wäschepflegezusammensetzung nach Anspruch 1, wobei $R^{a2}$ und $R^{b2}$ jeweils $C_{3-5}$-Alkyl sind, wobei $R^{a2}$ und $R^{b2}$ bevorzugt t-Butyl sind.

6. Wäschepflegezusammensetzung nach Anspruch 1, wobei $R^{a1}$ und $R^{b1}$ jeweils eine lineare $C_{1-8}$-Alkylgruppe sind, wobei $R^{a1}$ und $R^{b1}$ bevorzugt jeweils eine lineare $C_{1-4}$-Alkylgruppe sind, wobei $R^{a1}$ und $R^{b1}$ besonders bevorzugt jeweils Methyl sind oder $R^{a1}$ und $R^{b1}$ jeweils Ethyl sind.

7. Wäschepflegezusammensetzung nach Anspruch 1, wobei $L^1$ $-CH_2CH_2-$ oder $-CH_2CH_2CH_2-$ ist.

8. Wäschepflegezusammensetzung nach Anspruch 1, wobei $L^2$ $-CH_2CH_2-$ oder $-CH_2CH_2CH_2-$ ist.

9. Wäschepflegezusammensetzung nach Anspruch 1, wobei $x^1$ eine ganze Zahl von 4-25 ist.

10. Wäschepflegezusammensetzung nach Anspruch 1, wobei $x^2$ eine ganze Zahl von 2-25 ist.

11. Wäschepflegezusammensetzung nach Anspruch 1, wobei $R^{a3}$ und $R^{b3}$ jeweils H sind.

12. Wäschepflegezusammensetzung nach Anspruch 1, wobei das Molverhältnis der ersten Phenolverbindung zu der zweiten Phenolverbindung von 50:1 bis 1:50 beträgt.

13. Wäschepflegezusammensetzung nach Anspruch 1, ferner umfassend eine dritte Phenolverbindung mit der folgenden Formel:

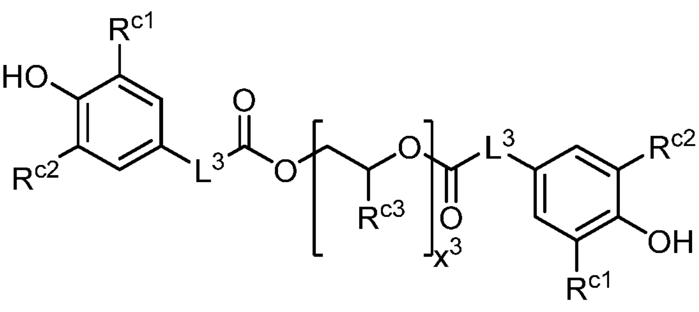

wobei $L^3$ ausgewählt ist aus Null, $-CH_2-$, $-CH_2CH_2-$ und $-CH_2CH_2CH_2-$,
$x^3$ eine ganze Zahl von 2-50 ist; vorausgesetzt, dass keine von $x^1$, $x^2$ und $x^3$ gleich sind;
$R^{c1}$ eine $C_{1-8}$-Alkylgruppe ist;
$R^{c2}$ eine $C_{1-8}$-Alkylgruppe ist; und
$R^{c3}$ H, $CH_3$ oder $CH_2CH_3$ ist;

bevorzugt ferner umfassend eine vierte Phenolverbindung mit der folgenden Formel:

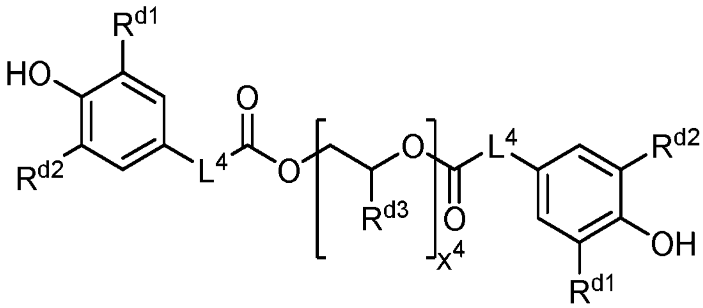

wobei $L^4$ ausgewählt ist aus Null, $-CH_2-$, $-CH_2CH_2-$ und $-CH_2CH_2CH_2-$,
$x^4$ eine ganze Zahl von 2-50 ist; vorausgesetzt, dass keine von $x^1$, $x^2$, $x^3$ und $x^4$ gleich sind;
$R^{d1}$ eine $C_{1-8}$-Alkylgruppe ist;
$R^{d2}$ eine $C_{1-8}$-Alkylgruppe ist; und
$R^{d3}$ H, $CH_3$ oder $CH_2CH_3$ ist.

**14.** Verfahren zum Waschen eines Artikels, umfassend Inkontaktbringen des Artikels mit der Zusammensetzung nach Anspruch 1, wobei der Artikel bevorzugt mit der Zusammensetzung in der Gegenwart von Wasser in Kontakt gebracht wird.

**15.** Verfahren zum Herstellen einer Wäschepflegezusammensetzung, umfassend Kombinieren eines wasserlöslichen Trägers, eines Tensids und einer Phenolzusammensetzung, wobei die Phenolzusammensetzung Folgendes umfasst:

eine erste Phenolverbindung mit der folgenden Formel:

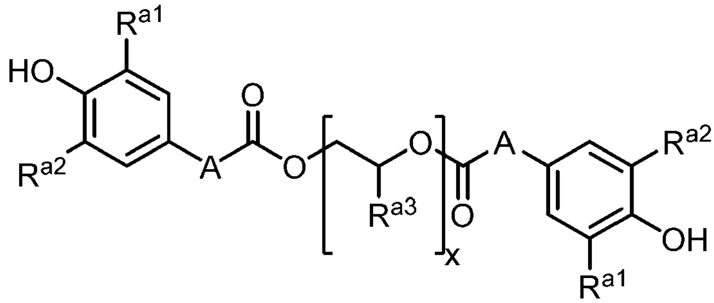

,

wobei A ausgewählt ist aus Null, $-CH_2-$, $-CH_2CH_2-$ und $-CH_2CH_2CH_2-$,
x eine ganze Zahl von 2-50 ist;
$R^{a1}$ eine $C_{1-8}$-Alkylgruppe ist;
$R^{a2}$ eine $C_{1-8}$-Alkylgruppe ist;
$R^{a3}$ H, $CH_3$ oder $CH_2CH_3$ ist; und

eine zweite Phenolverbindung mit der folgenden Formel:

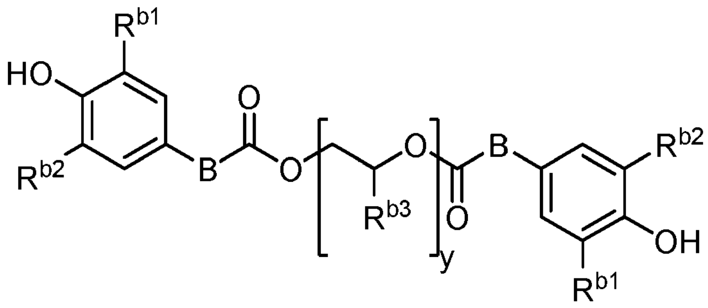

,

wobei B ausgewählt ist aus Null, $-CH_2-$, $-CH_2CH_2-$ und $-CH_2CH_2CH_2-$,
y eine ganze Zahl von 2-50 ist; vorausgesetzt, dass x und y nicht gleich sind;
$R^{b1}$ eine $C_{1-8}$-Alkylgruppe ist;
$R^{b2}$ eine $C_{1-8}$-Alkylgruppe ist; und
$R^{b3}$ H, $CH_3$ oder $CH_2CH_3$ ist.

## Revendications

**1.** Composition d'entretien du linge comprenant :

a) un tensioactif ; et
b) une composition phénolique comprenant :

i) un premier composé phénolique répondant à la formule :

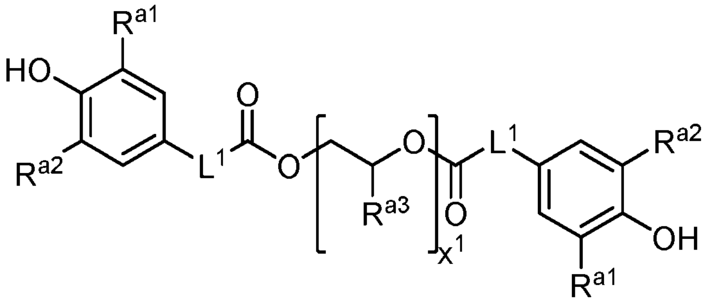

,

où $L^1$ est choisi parmi nul, $-CH_2-$, $-CH_2CH_2-$ et $-CH_2CH_2CH_2-$,
$x^1$ est un entier de 2 à 50 ;
$R^{a1}$ est un groupe alkyle en $C_{1-8}$ ;
$R^{a2}$ est un groupe alkyle en $C_{1-8}$ ;
$R^{a3}$ est H, $CH_3$ ou $CH_2CH_3$ ;

ii) un deuxième composé phénolique répondant à la formule :

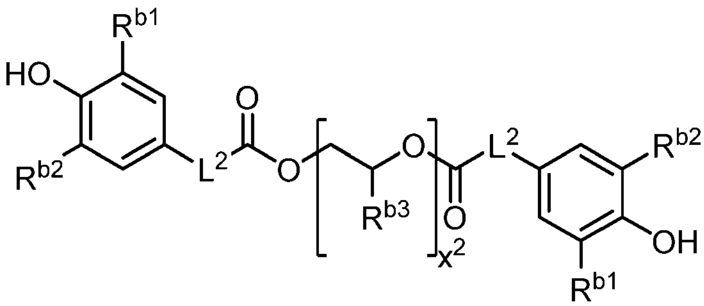

,

où $L^2$ est choisi parmi nul, $-CH_2-$, $-CH_2CH_2-$ et $-CH_2CH_2CH_2-$,
$x^2$ est un entier de 2 à 50 ; à condition que $x^1$ et $x^2$ ne soient pas identiques ;
$R^{b1}$ est un groupe alkyle en $C_{1-8}$ ;
$R^{b2}$ est un groupe alkyle en $C_{1-8}$ ; et
$R^{b3}$ est H, $CH_3$ ou $CH_2CH_3$.

**2.** Composition d'entretien du linge selon la revendication 1, dans laquelle la composition phénolique est présente en une quantité de 0,0001 % en poids à 1,0 % en poids par rapport au poids total de la composition d'entretien du linge.

**3.** Composition d'entretien du linge selon la revendication 1, dans laquelle $R^{a1}$ et $R^{a2}$ ne sont pas identiques.

**4.** Composition d'entretien du linge selon la revendication 1, dans laquelle $R^{b1}$ et $R^{a2}$ ne sont pas identiques.

**5.** Composition d'entretien du linge selon la revendication 1, dans laquelle $R^{a2}$ et $R^{b2}$ sont chacun un alkyle en $C_{3-5}$, de préférence dans laquelle $R^{a2}$ et $R^{b2}$ sont un t-butyle.

**6.** Composition d'entretien du linge selon la revendication 1, dans laquelle $R^{a1}$ et $R^{b1}$ sont chacun un groupe alkyle en $C_{1-8}$ linéaire, de préférence dans laquelle $R^{a1}$ et $R^{b1}$ sont chacun un groupe alkyle en $C_{1-4}$ linéaire, idéalement dans laquelle $R^{a1}$ et $R^{b1}$ sont chacun un méthyle, ou $R^{a1}$ et $R^{b1}$ sont chacun un éthyle.

**7.** Composition d'entretien du linge selon la revendication 1, dans laquelle $L^1$ est $-CH_2CH_2-$ ou $-CH_2CH_2CH_2-$.

**8.** Composition d'entretien du linge selon la revendication 1, dans laquelle $L^2$ est $-CH_2CH_2-$ ou $-CH_2CH_2CH_2-$.

**9.** Composition d'entretien du linge selon la revendication 1, dans laquelle $x^1$ est un entier de 4 à 25.

**10.** Composition d'entretien du linge selon la revendication 1, dans laquelle $x^2$ est un entier de 2 à 25.

**11.** Composition d'entretien du linge selon la revendication 1, dans laquelle $R^{a3}$ et $R^{b3}$ sont chacun H.

**12.** Composition d'entretien du linge selon la revendication 1, dans laquelle le rapport molaire du premier composé phénolique au deuxième composé phénolique est de 50:1 à 1:50.

**13.** Composition d'entretien du linge selon la revendication 1, comprenant en outre un troisième composé phénolique répondant à la formule :

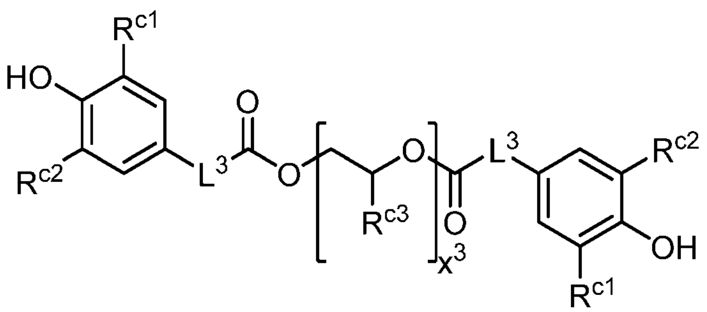

où $L^3$ est choisi parmi nul, $-CH_2-$, $-CH_2CH_2-$ et $-CH_2CH_2CH_2-$,
$x^3$ est un entier de 2 à 50 ; à condition qu'aucun de $x^1$, $x^2$ et $x^3$ ne soit identique ;
$R^{c1}$ est un groupe alkyle en $C_{1-8}$ ;
$R^{c2}$ est un groupe alkyle en $C_{1-8}$ ; et
$R^{c3}$ est H, $CH_3$ ou $CH_2CH_3$ ;

comprenant en outre de préférence un quatrième composé phénolique répondant à la formule :

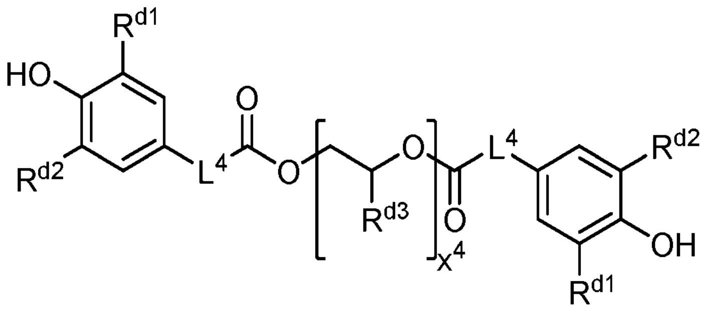

où $L^4$ est choisi parmi nul, $-CH_2-$, $-CH_2CH_2-$ et $-CH_2CH_2CH_2-$,
$x^4$ est un entier de 2 à 50 ; à condition qu'aucun de $x^1$, $x^2$, $x^3$ et $x^4$ ne soit identique ;
$R^{d1}$ est un groupe alkyle en $C_{1-8}$ ;
$R^{d2}$ est un groupe alkyle en $C_{1-8}$ ; et
$R^{d3}$ est H, $CH_3$ ou $CH_2CH_3$.

**14.** Procédé de lavage d'un article comprenant la mise en contact de l'article avec la composition selon la revendication 1, de préférence dans lequel l'article est mis en contact avec la composition en présence d'eau.

**15.** Procédé de fabrication d'une composition d'entretien du linge, comprenant la combinaison d'un support soluble dans l'eau, d'un tensioactif et d'une composition phénolique, ladite composition phénolique comprenant :

un premier composé phénolique répondant à la formule :

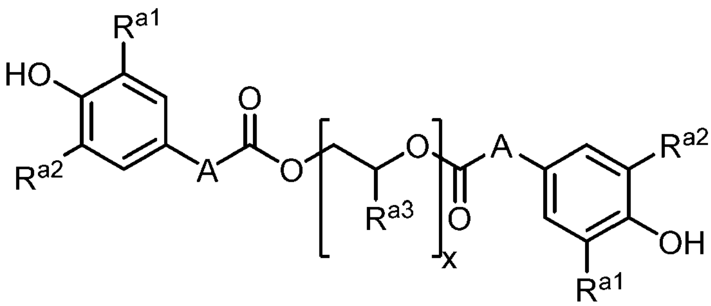

,

où A est choisi parmi nul, $-CH_2-$, $-CH_2CH_2-$ et $-CH_2CH_2CH_2-$,
x est un entier de 2 à 50 ;
$R^{a1}$ est un groupe alkyle en $C_{1-8}$ ;
$R^{a2}$ est un groupe alkyle en $C_{1-8}$ ;
$R^{a3}$ est H, $CH_3$ ou $CH_2CH_3$ ; et

un deuxième composé phénolique répondant à la formule :

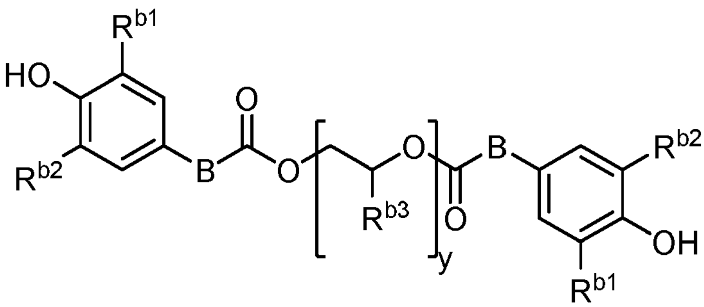

,

où B est choisi parmi nul, $-CH_2-$, $-CH_2CH_2-$ et $-CH_2CH_2CH_2-$,
y est un entier de 2 à 50 ; à condition que x et y ne soient pas identiques ;

$R^{b1}$ est un groupe alkyle en $C_{1-8}$;
$R^{b2}$ est un groupe alkyle en $C_{1-8}$ ; et
$R^{b3}$ est H, $CH_3$ ou $CH_2CH_3$.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2011130986 A
- US 10731112 B
- US 10876079 B
- US 5576282 A
- US 4915854 A
- US 4412934 A
- US 4634551 A
- US 5523434 A
- US 5998350 A
- US 5698504 A
- US 5695679 A
- US 5686401 A
- US 5686014 A
- US 5405412 A
- US 5405413 A
- US 5130045 A
- US 08064564 B
- US 4430243 A
- US 5718614 A
- US 5360569 A
- US 5442066 A
- US 5478357 A
- US 5370826 A
- US 5482515 A
- US 5550256 A
- WO 9513351 A
- WO 9513352 A
- WO 9513353 A
- US 4217105 A
- US 5916481 A
- EP 91202655
- US 68758204 P
- US 5759990 A
- US 20040204337 A1, Corona
- US 20040229769 A1, Smith
- US 6494920 B
- US 5574179 A


### Non-patent literature cited in the description


- **ZHANG et al.** *J Material Sci Eng*, vol. 6, 393
- Bleaching Agents (Survey). Kirk Othmer's Encyclopedia of Chemical Technology. John Wiley & Sons, 1992, vol. 4, 271-300